# EUROPEAN PATENT SPECIFICATION

(11) **EP 0 672 160 B1**
(45) Date of publication and mention of the grant of the patent: **11.08.2010**
(21) Application number: 94903405.2
(22) Date of filing: 02.12.1993
(51) Int. Cl.: C12N 15/90, A61K 48/00, C12N 15/18, C12N 15/27

(54) **ACTIVATING EXPRESSION OF AN AMPLIFYING ENDOGENOUS GENE BY HOMOLOGOUS RECOMBINATION**
EXPRIMIERUNGSAKTIVATION UND VERVIELFÄLTIGUNG VON ENDOGENEM GEN MIT HOMOLOGER REKOMBINATION
ACTIVATION D'EXPRESSION ET D'AMPLIFICATION D'UN GENE ENDOGENE PAR RECOMBINAISON HOMOLOGUE

(30) Priority: 03.12.1992 US 985586
(43) Date of publication of application: 20.09.1995
(73) Proprietor: TRANSKARYOTIC THERAPIES, INC., Cambridge, MA 02139 (US)
(72) Inventor: TRECO, Douglas, A., Arlington, MA 02174 (US); HEARTLEIN, Michael, W., Boxborough, MA 01719 (US); SELDEN, Richard, F., Wellesley, MA 02181 (US)
(74) Representative: White, Martin Paul
(86) International application number: PCT/US1993/011704
(87) International publication number: WO 1994/012650

(56) References cited:
- EP-A- 0 117 059
- WO-A-90/12025
- WO-A-91/00361
- WO-A-91/09955
- WO-A-92/10561
- WO-A-92/20808
- US-A- 4 822 736

## Description

### Background of the Invention

Current approaches to treating disease by administering therapeutic proteins include ia vitro production of therapeutic proteins for conventional pharmaceutical delivery (e.g. intravenous, subcutaneous, or intramuscular injection) and, more recently, gene therapy.

Proteins of therapeutic interest are generally produced by introducing exogenous DNA encoding the protein of therapeutic interest into appropriate cells. Presently- available approaches to gene therapy make use of infectious vectors, such as retroviral vectors, which include the genetic material to be expressed. Such approaches have limitations, such as the potential of generating replication-competent virus during vector production; recombination between the therapeutic virus and endogenous retroviral genomes, potentially generating infectious agents with novel cell specificities, host ranges, or increased virulence and cytotoxicity; independent integration into large numbers of cells, increasing the risk of a tumorigenic insertional event; limited cloning capacity in the retrovirus (which restricts therapeutic applicability) and short-lived in vivo expression of the product of interest. A better approach to providing gene products, particularly one which avoids the risks associated with presently available methods and provides long-term treatment, would be valuable.

WO 91/09955 discloses that normally transcriptionally silent genes in a cell line or microorganism may be activated for expression by inserting a DNA regulatory element which is capable of promoting the expression of a normally expressed gene product in that cell or which is promiscuous, the regulatory element being inserted so as to be operatively linked with the normally silent gene in question. The insertion is accomplished by means of homologous recombination by creating a DNA construct including a segment having a DNA segment of the normally silent gene (targeting DNA) and the DNA regulatory element to induce gene transcription. The technique is also used to modify the expression characteristics of any endogenous gene of a given cell line or microorganism.

WO 92/20808 discloses methods for modification of genomic target sites, where desired changes that can be small or subtle may be introduced into the target site to provide for modification of target genes or regulatory sequences. A marker may be retained without interference with the functioning of a target gene or select for excision of exogenous DNA to leave a single copy of the target gene with the modification.

WO 93/09222 relates to transfected primary and secondary somatic cells of vertebrate origin, particularly mammalian origin, transfected with exogenous genetic material (DNA) which encodes a desired (e.g. a therapeutic) product or is itself a desired (e.g. a therapeutic) product. It also discloses methods by which primary and secondary cells are transfected to include exogenous genetic material, including DNA targeting by homologous recombination, methods of producing clonal cell strains or heterogenous cell strains, methods of gene therapy in which the transfected primary or secondary cells are used, and methods of producing antibodies using the transfected primary or secondary cells.

### Summary of the Invention

The present invention relates to an in vitro gene amplification method as set forth in claims to 18. This allows the in vitro production of therapeutic proteins. It provides cells and constructs that can be used for the production and delivery of therapeutic proteins by gene therapy. The present method describes an approach which can activate expression of endogenous cellular genes, and further allows amplification of the activated endogenous cellular genes, which does not require in vitro manipulation and transfection of exogenous DNA encoding proteins of therapeutic interest.

The present invention relates to transfected cells within the scope of claim 20. Transfected primary or secondary cells (i.e., non-immortalized cells) and transfected immortalized cells are useful for producing proteins, particularly therapeutic proteins. Methods of making such cells and of using the cells for in vitro protein production are set forth in claims 1 to 19. They can be used in medicine and gene therapy. Cells of the present invention are of vertebrate origin, particularly of mammalian origin and even more particularly of human origin. Cells produced by the method of the present invention contain exogenous DNA which encodes a therapeutic product, exogenous DNA which is itself a therapeutic product and/or exogenous DNA which causes the transfected cells to express a gene at a higher level or with a pattern of regulation or induction that is different than occurs in the corresponding nontransfected cell.

The cells may be primary, secondary, or immortalized cells that are transfected to include exogenous genetic material. They may be clonal cell strains or heterogenous cell strains. They may be used for immunizing animals, or producing antibodies in immunized animals.

The present invention relates particularly to a method of gene targeting or homologous recombination in cells of vertebrate, particularly mammalian, origin. That is, it relates to a method of introducing DNA into primary, secondary, or immortalized cells of vertebrate origin through homologous recombination, such that the DNA is introduced into genomic DNA of the primary, secondary, or immortalized cells at a preselected site. The targeting sequences used are determined by (selected with reference to) the site into which the exogenous DNA is to be inserted. The present invention further relates to homologously recombinant primary, secondary, or immortalized cells, referred to as homologously recombinant (HR) primary, secondary or immortalized cells, produced by the present method and to uses of the HR primary, secondary, or immortalized cells.

The present invention can be used for activating (i.e., turning on) a gene present in primary, secondary, or immortalized cells of vertebrate origin, which is normally not expressed in the cells or is not expressed at physiologically significant levels in the cells as obtained. Homologous recombination can be used to replace or disable the regulatory region normally associated with the gene in cells as obtained with a regulatory sequence which causes the gene to be expressed at levels higher than evident in the corresponding nontransfected cell, or to display a pattern of regulation or induction that is different than evident in the corresponding nontransfected cell. The present invention, therefore, can be used in a method of making proteins by turning on or activating an endogenous gene which encodes the desired product in transfected primary, secondary, or immortalized cells.

The activated gene is amplified by the inclusion of a selectable marker gene which has the property that cells containing amplified copies of the selectable marker gene can be selected for by culturing the cells in the presence of the appropriate selectable agent. The activated endogenous gene which is near or linked to the amplified selectable marker gene will also be amplified in cells containing the amplified selectable marker gene. Cells containing many copies of the activated endogenous gene are useful for in vitro protein production and gene therapy.

Gene targeting and amplification as disclosed in the present invention are particularly useful for turning on the expression of genes which form transcription units which are sufficiently large that they are difficult to isolate and express, or for turning on genes for which the entire protein coding region is unavailable or has not been cloned.

Transfected cells of the present invention are useful in a number of applications in humans and animals. In one embodiment, the cells can be implanted into a human or an animal for protein delivery in the human or animal. For example, human growth hormone (hGH), human EPO (hEPO), human insulinotropin and other proteins can be delivered systemically or locally in humans for therapeutic benefits. Barrier devices, which contain transfected cells which express a therapeutic product and through which the therapeutic product is freely permeable, can be used to retain cells in a fixed position in vivo or to protect and isolate the cells from the host's immune system. Barrier devices are particularly useful and allow transfected immortalized cells, transfected cells from another species (transfected xenogeneic cells), or cells from a nonhistocompatibility-matched donor (transfected allogeneic cells) to be implanted for treatment of human or animal conditions or for agricultural uses (e.g., meat and dairy production). Barrier devices also allow convenient short-term (i.e., transient) therapy by providing ready access to the cells for removal when the treatment regimen is to be halted for any reason. Transfected xenogeneic and allogeneic cells may be used for short-term gene therapy, such that the gene product produced by the cells will be delivered in vivo until the cells are rejected by the host's immune system.

Transfected cells of the present invention are also useful for eliciting antibody production or for immunizing humans and animals against pathogenic agents. Implanted transfected cells can be used to deliver immunizing antigens that result in stimulation of the host's cellular and humoral immune responses. These immune responses can be designed for protection of the host from future infectious agents (i.e., for vaccination), to stimulate and augment the disease-fighting capabilities directed against an ongoing infection, or to produce antibodies directed against the antigen produced in vivo by the transfected cells that can be useful for therapeutic or diagnostic purposes. Removable barrier devices can be used to allow a simple means of terminating exposure to the antigen. Alternatively, the use of cells that will ultimately be rejected (xenogeneic or allogeneic transfected cells) can be used to limit exposure to the antigen, since antigen production will cease when the cells have been rejected.

The methods of the present invention can be used to produce primary, secondary, or immortalized cells producing a wide variety of therapeutically useful products, including (but not limited to): hormones, cytokines, antigens, antibodies, enzymes, clotting factors, transport proteins, receptors, regulatory proteins, structural proteins, transcription factors, or anti-sense RNA. Additionally, the methods of the present invention can be used to produce cells which produce non-naturally occurring ribozymes, proteins, or nucleic acids which are useful for in vitro production of a therapeutic product or for gene therapy.

### Brief Description of the Drawings

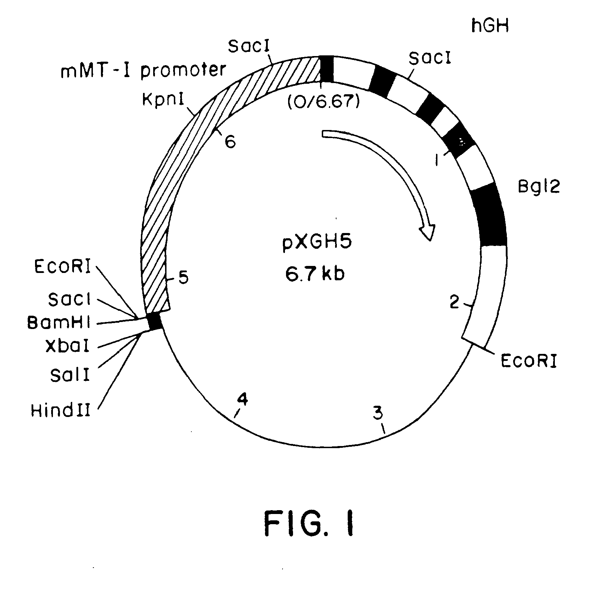

Figure 1 is a schematic representation of plasmid pXGH5, which includes the human growth hormone (hGH) gene under the control of the mouse metallothionein promoter.

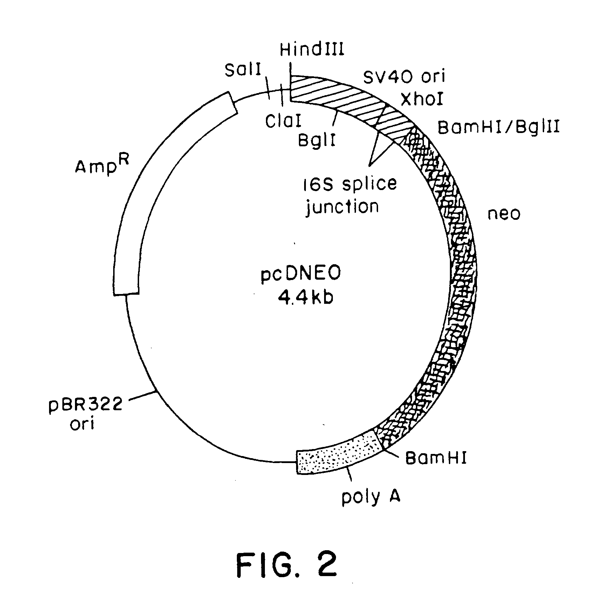

Figure 2 is a schematic representation of plasmid pcDNEO, which includes the neo coding region (BamHI-BglII fragment) from plasmid pSV2neo inserted into the BamHI site of plasmid pcD; the Amp-R and pBR322Ori sequences from pBR322; and the polyA, 16S splice junctions and early promoter regions from SV40.

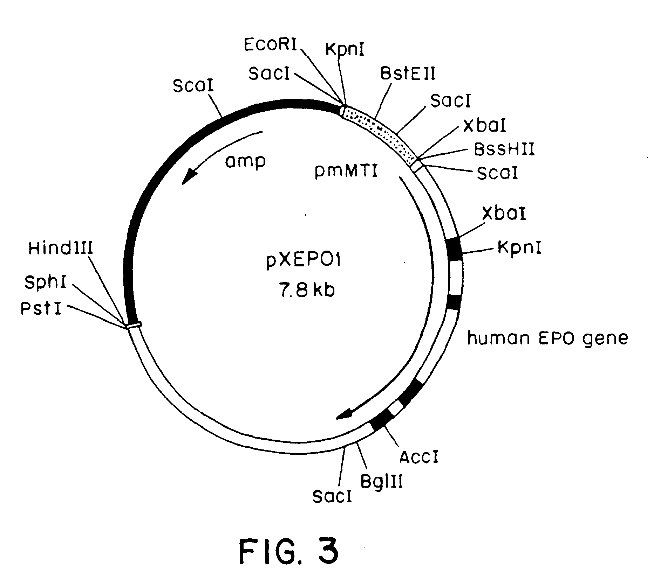

Figure 3 is a schematic representation of plasmid pXEPO1. The solid black arc represents the pUC12 backbone and the arrow denotes the direction of transcription of the ampicillin resistance gene. The stippled arc represents the mouse metallothionein promoter (pmMT1). The unfilled arc interrupted by black boxes represents the human erythropoietin EPO gene (the black boxes denote exons and the arrow indicates the direction hEPO transcription). The relative positions of restriction endonuclease recognition sites are indicated.

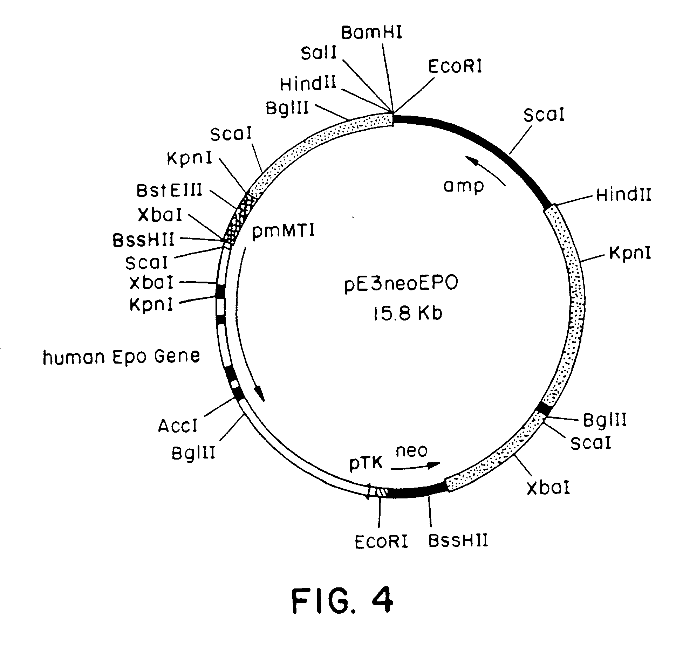

Figure 4 is a schematic representation of plasmid pE3neoEPO. The positions of the human erythropoietin gene and the neo and amp resistance genes are indicated. Arrows indicate the directions of transcription of the various genes. pmMT1 denotes the mouse metallothionein promoter (driving hEPO expression) and pTK denotes the Herpes Simplex Virus thymidine kinase promoter (driving neo expression). The dotted regions of the map mark the positions of human HPRT sequences. The relative positions of restriction endonuclease recognition sites are indicated.

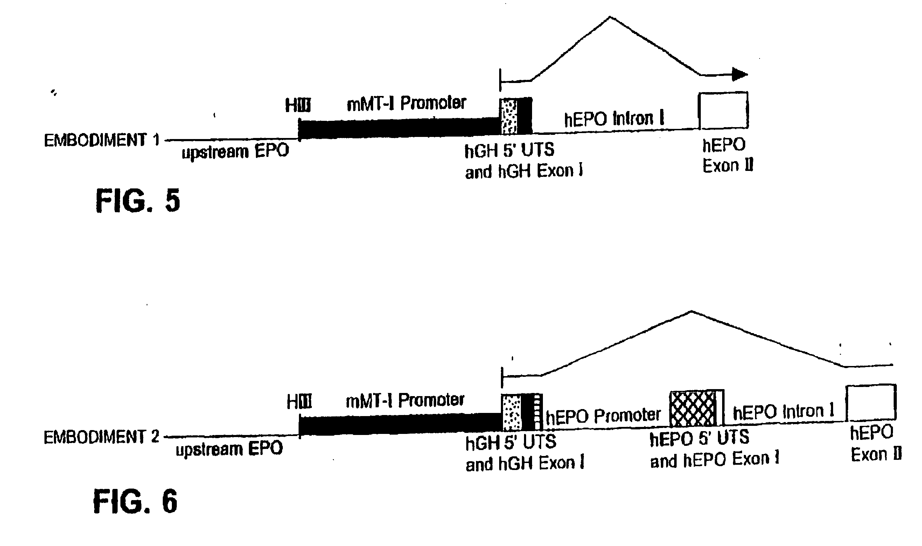

Figure 5 is a schematic diagram of a strategy for transcriptionally activating the hEPO gene; the thin lines represent hEPO sequences; thick lines, mouse metallothionein I promoter; stippled box, 5' untranslated region of hGH; solid box, hGH exon 1; open boxes, hEPO coding sequences; HIII, HindIII site.
Figure 6 is a schematic diagram of a strategy for transcriptionally activating the hEPO gene; the thin lines represent hEPO sequences; thick lines, mouse metallothionein I promoter; stippled box, 5' untranslated region of hGH; solid box, hGH exon 1; striped box, 10 bp linker from hEPO intron 1; cross-hatched box, 5' untranslated region of hEPO; and open boxes, hEPO coding sequences; HIII, HindIII site.

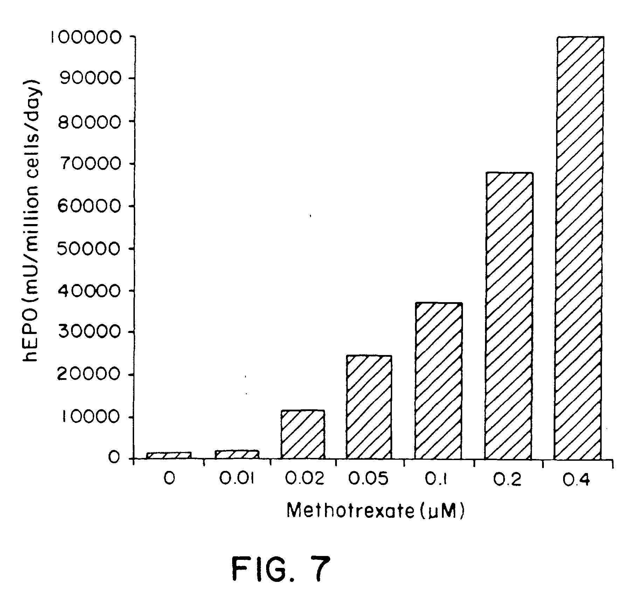

Figure 7 is a graphic representation of erythropoietin expression in a targeted human cell line subjected to stepwise selection in 0.02, 0.05, 0.1, 0.2 and 0.4 µM methotrexate.

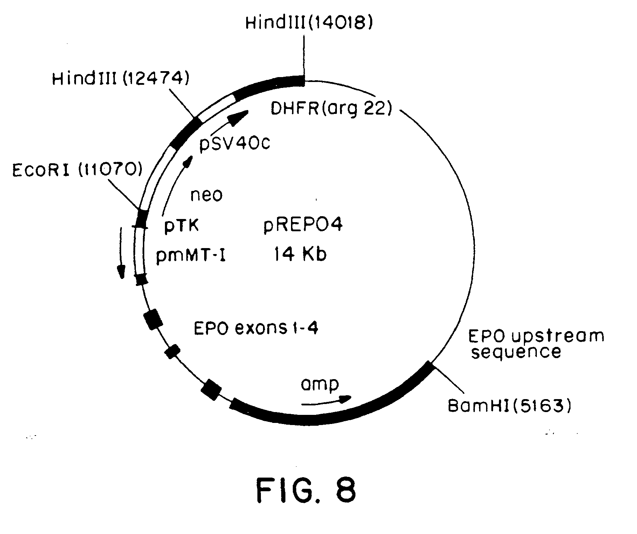

Figure 8 is a schematic representation of plasmid pREPO4.

### Detailed Description of the Invention

### Overview of the Invention

The present invention is set forth in claims 1 to 28.

It allows clinically useful products in physiologically useful quantities to be provided to an individual in need thereof through the use of transfected cells of the present invention. The cells can also be used for vaccinating animals for protection against pathogenic viruses or microbial agents expressing epitopes antigenically related to products expressed by the transfected cells. Antibodies can be produced directed against a product made by the transfected primary, secondary, or immortalized cells. Clinically useful products can be produced in vitro, by purification from the transfected cells, or produced in vivo, by implantation into a non-human animal or human (i.e., gene therapy). Whether produced in vitro or in vivo, the clinically useful products can include hormones, cytokines, antigens, antibodies, enzymes, clotting factors, transport proteins, receptors, regulatory proteins, structural proteins, transcription factors, anti-sense RNA. Additionally, the methods of the present invention can be used to produce cells which produce non-naturally occurring ribozymes, proteins, or nucleic acids.

In one embodiment, the present invention relates to a method of gene or DNA targeting in cells of vertebrate, particularly mammalian, origin. That is, it relates to a method of introducing DNA into primary, secondary, or immortalized cells of vertebrate origin through homologous recombination or targeting of the DNA, which is introduced into genomic DNA of the primary, secondary, or immortalized cells at a preselected site. The targeting sequences used are determined by (selected with reference to) the site into which the exogenous DNA is to be inserted. The present invention further relates to homologously recombinant primary, secondary or immortalized cells, referred to as homologously recombinant (HR) primary, secondary or immortalized cells, produced by the present method and to uses of the HR primary, secondary, or immortalized cells.

The present invention can be used for activating a gene which is present in primary cells, secondary cells or immortalized cells of vertebrate origin, but is normally not expressed in the cells or is not expressed at significant levels in the cells. Homologous recombination or targeting can be used to replace or disable the regulatory region normally associated with the gene with a regulatory sequence which causes the gene to be expressed at levels higher than evident in the corresponding nontransfected cell, or causes the gene to display a pattern of regulation or induction that is different than evident in the corresponding nontransfected cell. The present invention, therefore, can be used for making proteins by activating an endogenous gene which encodes the desired product in transfected primary, secondary or immortalized cells.

The present invention can be used for activating (i.e turning on) and amplifying an endogenous gene encoding a desired product in a transfected, primary, secondary, or immortalized cell. That is, it can be used for introducing, by homologous recombination with genomic DNA, DNA sequences which are not normally functionally linked to the endogenous gene and (1) which, when inserted into the host genome at or near the endogenous gene, serve to alter (e.g., activate) the expression of the endogenous gene, and further (2) allow for selection of cells in which the activated endogenous gene is amplified. Amplifiable DNA sequences useful in the present invention include, but are not limited to, sequences which encode the selectable markers dihydrofolate reductase, adenosine deaminase, and the CAD gene (encoding the trifunctional protein carbamyl phosphate synthase, aspartate transcarbamylase, and dihydro-orotase). Improved versions of these sequences and other amplifiable sequences can also be used. The amplifiable DNA sequences encoding a selectable marker and the DNA sequences which alter the regulation of expression of the endogenous gene can be introduced into the primary, secondary, or immortalized cell in association with DNA sequences homologous to genomic DNA sequences at a preselected site in the cell's genome. This site will generally be within or upstream of a gene encoding a therapeutic product or at a site that affects the desired gene's function. The DNA sequences which alter the expression of the endogenous gene, the amplifiable sequences which encode a selectable marker, and the sequences which are homologous to a preselected site in genomic DNA can be introduced into the primary, secondary, or immortalized cell as a single DNA construct, or as separate DNA sequences which become physically linked in the genome of a transfected cell. Further, the DNA can be introduced as linear, double stranded DNA, with or without single stranded regions at one or both ends, or the DNA can be introduced as circular DNA. After the exogenous DNA is introduced into the cell, the cell is maintained under conditions appropriate for homologous recombination to occur between the genomic DNA and a portion of the introduced DNA. Homologous recombination between the genomic DNA and the introduced DNA results in a homologously recombinant primary, secondary, or immortalized cell in which sequences which alter the expression of an endogenous gene, and the amplifiable sequences encoding a selectable marker, can be operatively linked to an endogenous gene encoding a therapeutic product. Culturing the resulting homologously recombinant cell under conditions which select for amplification of the amplifiable DNA encoding a selectable marker results in a cell containing an amplified selectable marker and a coamplified endogenous gene whose expression has been altered. Cells produced by this method can be cultured under conditions suitable for the expression of the therapeutic protein, thereby producing the therapeutic protein in vitro, or the cells can be used for in vivo delivery of a therapeutic protein (i.e., gene therapy).

Additional embodiments are possible. The targeting event can be a simple insertion of a regulatory sequence, placing the endogenous gene under the control of the new regulatory sequence (for example, by insertion of either a promoter or an enhancer, or both, upstream of an endogenous gene). The targeting event can be a simple deletion of a regulatory element, such as the deletion of a tissue-specific negative regulatory element. The targeting event can replace an existing element; for example, a tissue-specific enhancer can be replaced by an enhancer that has broader or different cell-type specificity than the naturally-occurring elements, or displays a pattern of regulation or induction that is different from the corresponding nontransfected cell. In this embodiment the naturally occurring sequences are deleted and new sequences are added. In all cases, the identification of the targeting event can be facilitated by the use of one or more selectable marker genes that are physically associated with the targeting DNA, allowing for the selection of cells in which the exogenous DNA has integrated into the host cell genome. The identification of the targeting event can also be facilitated by the use of one or more marker genes exhibiting the property of negative selection, such that the negatively selectable marker is linked to the exogenous DNA, but configured such that the negatively selectable marker flanks the targeting sequence, and such that a correct homologous recombination event with sequences in the host cell genome does not result in the stable integration of the negatively selectable marker. Markers useful for this purpose include the Herpes Simplex Virus thymidine kinase (TK) gene or the bacterial xanthine-guanine phosphoribosyl-transferase (gpt) gene.

### Transfected Cells

As used herein, the term primary cell includes cells present in a suspension of cells isolated from a vertebrate tissue source (prior to their being plated, i.e., attached to a tissue culture substrate such as a dish or flask), cells present in an explant derived from tissue, both of the previous types of cells plated for the first time, and cell suspensions derived from these plated cells. The term secondary cell or cell strain refers to cells at all subsequent steps in culturing. That is, the first time a plated primary cell is removed from the culture substrate and replated (passaged), it is referred to herein as a secondary cell, as are all cells in subsequent passages. Secondary cells are cell strains which consist of secondary cells which have been passaged one or more times. A cell strain consists of secondary cells that: 1) have been passaged one or more times; 2) exhibit a finite number of mean population doublings in culture; 3) exhibit the properties of contact-inhibited, anchorage dependent growth (anchorage-dependence does not apply to cells that are propagated in suspension culture); and 4) are not immortalized.

Cells transfected by the subject method have the expression of an endogenous gene altered as described in claim 1. This includes four types or categories: 1) cells which do not, as obtained, make or contain the therapeutic product, 2) cells which make or contain the therapeutic product but in smaller quantities than normal (in quantities less than the physiologically normal lower level) or in defective form, 3) cells which make the therapeutic product at physiologically normal levels, but are to be augmented or enhanced in their content or production, and 4) cells in which it is desirable to change the pattern of regulation or induction of a gene encoding a therapeutic product.

Primary and secondary cells to be transfected by the present method can be obtained from a variety of tissues and include all cell types which can be maintained in culture. For example, primary and secondary cells which can be transfected by the present method include fibroblasts, keratinocytes, epithelial cells (e.g., mammary epithelial cells, intestinal epithelial cells), endothelial cells, glial cells, neural cells, formed elements of the blood (e.g., lymphocytes, bone marrow cells), muscle cells and precursors of these somatic cell types. Primary cells are preferably obtained from the individual to whom the transfected primary or secondary cells are administered. However, primary cells can be obtained from a donor (other than the recipient) of the same species or another species (e.g., mouse, rat, rabbit, cat, dog, pig, cow, bird, sheep, goat, horse).

Transfected primary and secondary cells have been produced, with or without phenotypic selection and shown to express exogenous DNA encoding a therapeutic product including, for example, hGH, EPO and insulinotropin.

Immortalized cells can also be transfected by the present method and used for either protein production or gene therapy. Examples of immortalized human cell lines useful for protein production or gene therapy by the present method include, but are not limited to, HT1080, HeLa, MCF-7 breast cancer cells, K-562 leukemia cells, KB carcinoma cells, 2780AD ovarian carcinoma cells, Raji (ATCC CCL 86) cells, Jurkat (ATCC TIB 152) cells, Namalwa (ATCC CRL 1432) cells, HL-60 (ATCC CCL 240) cells, Daudi (ATCC CCL 213) cells, RPMI 8226 (ATCC CCL 155) cells and MOLT-4 (ATCC CRL 1582) cells. Immortalized cells from other species (e.g., chinese hamster ovary (CHO) cells or mouse L cells) can be used for in vitro protein production or gene therapy. In addition, primary or secondary human cells, as well as primary or secondary cells from other species which display the properties of gene amplification in vitro can be used for in vitro protein production or gene therapy.

### Exogenous DNA

Exogenous DNA incorporated into primary, secondary or immortalized cells by the present method is: as described in part a) of claim 1 1) DNA which encodes a translation or transcription product whose expression in cells is desired, or a portion of a translation or transcription product, such as a protein product or RNA product useful to treat an existing condition or prevent it from occurring (eg., hGH, EPO or insulinotropin); or 2) DNA which does not encode a gene product but is itself useful, such as a transcriptional regulatory sequence or DNA useful to treat an existing condition or prevent it from occurring.

DNA transfected into primary, secondary or immortalized cells can encode an entire desired product, or can encode, for example, the active or functional portion(s)

It alters the expression of a gene product in a cell. The product can be, for example, a hormone, a cytokine, an antigen, an antibody, an enzyme, a clotting factor, a transport protein, a receptor, a regulatory protein, a structural protein, a transcription factor, an anti-sense RNA, or a ribozyme. Additionally, the product can be a protein or a nucleic acid which does not occur in nature (i.e., a novel protein or novel nucleic acid). The DNA can be obtained from a source in which it occurs in nature or can be produced, using genetic engineering techniques or synthetic processes. The DNA can alter the expression of one or more therapeutic products. After transfection, the exogenous DNA is stably incorporated into the recipient cell's genome (along with any additional sequences present in the DNA construct used), from which it is expressed or otherwise functions. Alternatively, the exogenous DNA can be used to target to DNA that exists episomally within cells.

DNA can be introduced into cells under the control of an inducible promoter, with the result that cells as produced or as introduced into an individual do not alter expression of the product but can be induced to do so (i.e., production is induced after the transfected cells are produced but before implantation or after implantation). DNA altering expression of a desired product can, of course, be introduced into cells in such a manner that expression is altered upon introduction (i.e., without induction).

As taught herein, gene targeting can be used to replace a gene's existing regulatory region with a regulatory sequence isolated from a different gene or a novel regulatory sequence synthesized by genetic engineering methods. Such regulatory sequences can be comprised of promoters, enhancers, scaffold-attachment regions, negative regulatory elements, transcriptional initiation sites, regulatory protein binding sites or combinations of said sequences. (Alternatively, sequences which affect the structure or stability of the RNA or protein produced can be replaced, removed, added, or otherwise modified by targeting, these sequences including polyadenylation signals, mRNA stability elements, splice sites, leader sequences for enhancing or modifying transport or secretion properties of the protein, or other sequences which alter or improve the function or stability of protein or RNA molecules). Introduction of the exogenous DNA can result in disablement of the endogenous sequences which control expression of the endogenous gene, either by replacing all or a portion of the endogenous (genomic) sequence or otherwise disrupting the function of the endogenous sequence. In the situation where targeting is used to replace a protein coding domain, chimeric, multifunctional proteins can be produced which combine structural, enzymatic, or ligand or receptor binding properties from two or more proteins into one polypeptide.

### Selectable Markers

A variety of selectable markers can be incorporated into primary, secondary or immortalized cells. For example, a selectable marker which confers a selectable phenotype such as drug resistance, nutritional auxotrophy, resistance to a cytotoxic agent or expression of a surface protein, can be used. Selectable marker genes which can be used include neo, gpt, dhfr, ada, pac, hyg, CAD, and hisD. The selectable phenotype conferred makes it possible to identify and isolate recipient cells. Amplifiable genes encoding selectable markers (e.g., ada, dhfr and the multifunctional CAD gene which encodes carbamyl phosphate synthase, aspartate transcarbamylase, and dihydro-orotase) have the added characteristic that they enable the selection of cells containing amplified copies of the selectable marker inserted into the genome. This feature provides a mechanism for significantly increasing the copy number of an adjacent or linked gene for which amplification is desirable.

Selectable markers can be divided into two categories: positively selectable and negatively selectable (in other words, markers for either positive selection or negative selection). In positive selection, cells expressing the positively selectable marker are capable of surviving treatment with a selective agent (such as neo, gpt, dhfr, ada, pac, hyg, mdrl and hisD). In negative selection, cells expressing the negatively selectable marker are destroyed in the presence of the selective agent (e.g., tk, gpt).

### DNA Constructs

DNA constructs, which include exogenous DNA and for use in the present invention have the features of part a) of claim 1. The DNA constructs may be introduced into cells by a variety of methods, including electroporation, microinjection, calcium phosphate precipitation, and liposome- polybrene- or DEAE dextran-mediated transfection. Alternatively, infectious vectors, such as retroviral, herpes, adeno-virus, adenovirus-associated, mumps and poliovirus vectors, can be used to introduce the DNA.

The DNA construct includes exogenous DNA and one or more targeting sequences, generally located at both ends of the exogenous DNA sequence. Targeting sequences are DNA sequences normally present in the genome of the cells as obtained (e.g., an essential gene, a nonessential gene or noncoding DNA, or sequences present in the genome through a previous modification). Such a construct is useful to integrate exogenous DNA (at a preselected site in a recipient cell is genome) that can be used to alter the expression of encoding a therapeutic product, such as a hormone, a cytokine, an antigen, an antibody, an enzyme, a clotting factor, a transport protein, a receptor, a regulatory protein, a structural protein, an anti-sense RNA, a ribozyme or a protein or a nucleic acid which does not occur in nature. In particular, exogenous DNA can alter the expression of one of the following: Factor VIII, Factor IX, erythropoietin, alpha-1 antitrypsin, calcitonin, glucocerebrosidase, growth hormone, low density lipoprotein (LDL) receptor, apolipoproteins (e.g. apolipoprotein E or apolipoprotein A-I), IL-2 receptor and its antagonists, insulin, globin, immunoglobulins, catalytic antibodies, the interleukins, insulin-like growth factors, superoxide dismutase, immune responder modifiers, parathyroid hormone, interferons, nerve growth factors, tissue plasminogen activators, and colony stimulating factors, and variants of these proteins which have improved or novel biological properties or more desirable half-life or turnover times in vivo. Such a construct is also useful to integrate exogenous DNA (at a preselected site in a recipient cell's genome) which is a therapeutic product, such as DNA sequences sufficient for sequestration of a protein or nucleic acid in the transfected primary or secondary cell, DNA sequences which bind to a cellular regulatory protein, DNA sequences which alter the secondary or tertiary chromosomal structure and DNA sequences which are transcriptional regulatory elements into genomic DNA of primary or secondary cells.

A construct of the present invention is useful to knock out, replace or repair a resident DNA sequence, such as an entire gene, a gene portion, a regulatory element or portion thereof or regions of DNA which, when removed, place regulatory and structural sequence in functional proximity. It is also useful for providing a marker useful for selection or amplification of linked sequences.

One or more selectable markers are included in the construct, which makes selection based on a selectable phenotype possible. Cells that stably integrate the construct will survive treatment with the selective agent; a subset of the stably transfected cells will be HR cells, which can be identified by a variety of techniques, including PCR, Southern hybridization and phenotypic screening.

If two targeting sequences and two selectable markers are present, the order of these components in the DNA construct can be: targeting sequence-selectable marker 1 - targeting sequence - selectable marker 2. In this embodiment selectable marker 2 displays the property of negative selection. That is, the gene product of selectable marker 2 can be selected against by growth in an appropriate media formulation containing an agent (typically a drug or metabolite analog) which kills cells expressing selectable marker 2. Recombination between the targeting sequences flanking selectable marker 1 with homologous sequences in the host cell genome results in the targeted integration of selectable marker 1, while selectable marker 2 is not integrated. Such recombination events generate cells which are stably transfected with selectable marker 1 but not stably transfected with selectable marker 2, and such cells can be selected for by growth in the media containing the selective agent which selects for selectable marker 1 and the selective agent which selects against selectable marker 2.

The DNA construct can include a positively selectable marker that allows for the selection of cells containing amplified copies of that marker. The amplification of such a marker results in the co-amplification of flanking DNA sequences. In this embodiment, the order of the aforesaid construct components is, for example: targeting sequences - DNA encoding an amplifiable positively selectable marker - DNA encoding a second selectable marker (optional) - DNA sequences corresponding to either an exogenous gene to be expressed under the control of a suitable promoter or a promoter only which is positioned to activate an endogenous gene - targeting DNA sequences.

A DNA construct can include an inducible promoter which controls expression, making inducible expression possible. Optionally, the DNA construct can include a bacterial origin of replication and bacterial antibiotic resistance markers, which allow for large-scale plasmid propagation in bacteria. A DNA construct which includes DNA encoding a selectable marker, along with additional sequences, such as a promoter, polyadenylation site and splice junctions, can be used to confer a selectable phenotype upon transfected primary or secondary cells (e.g., plasmid pcDNEO, schematically represented in Figure 2). Such a DNA construct can be co-transfected into primary or secondary cells, along with a targeting DNA sequence.

### Uses of Transfected Cells

Cells produced using the methods and DNA constructs described herein can be used for a wide variety of purposes. Primary, secondary, or immortalized cells of vertebrate origin can be produced in which 1) DNA already present in a recipient cell is repaired, altered, deleted, or replaced; 2) a gene or DNA sequence which encodes a therapeutic product (or other desired product) or is itself a therapeutic product is introduced into the genome of a recipient cell at a preselected site (i.e gene targeting); 3) regulatory sequences present in the primary, secondary or immortalized cell recipients have been repaired, altered, deleted or replaced; or 4) an entire gene or gene portion has been repaired, altered, deleted, or replaced. Homologous recombination can also be used to produce universal donor cells, in which cell surface markers involved in histocompatibility have been altered, deleted or replaced, or in which the expression of such markers is altered, impaired, or eliminated.

The cells of the present invention are useful for in vitro production of therapeutic products which can be purified and delivered by conventional pharmaceutic routes. For example, primary, secondary, or immortalized human cells can be transfected with exogenous DNA containing a regulatory region which, upon homologous recombination with genomic DNA sequences, results in the replacement of an endogenous target gene's regulatory region with a regulatory region that allows novel expression and/or regulation of the target gene and, ultimately, production of a therapeutically useful product by the transfected cell. The activated endogenous target gene can further be amplified via a selectable marker gene is included in the targeting DNA. With or without amplification, these cells can be subject to large-scale cultivation for harvest of intracellular or extracellular protein products.

Transfected cells of the present invention are useful, as populations of transfected primary cells, transfected clonal cell strains, transfected heterogenous cell strains, and as cell mixtures in which at least one representative cell of one of the three preceding categories of transfected cells is present, as a delivery system for treating an individual with an abnormal or undesirable condition which responds to delivery of a therapeutic product, which is either: 1) a therapeutic protein (e.g., a protein which is absent, underproduced relative to the individual's physiologic needs, defective or inefficiently Transfected primary cells, clonal cell strains or heterogenous cell strains can be administered to an individual in whom the abnormal or undesirable condition is to be treated or prevented, in sufficient quantity and by an appropriate route, to express or make available the exogenous DNA at physiologically relevant levels. A physiologically relevant level is one which either approximates the level at which the product is produced in the body or results in improvement of the abnormal or undesirable condition. By using the present invention regulatory sequence, can be introduced into a preselected site in genomic DNA through homologous recombination and function to cause recipient cells to produce a product which is normally not expressed in the cells or to produce the product of a higher level than occurs in the corresponding nontransfected cell. When the regulatory sequence (e.g., a promoter) is introduced, it can replace or disable a regulatory sequence normally associated with a gene, and can result in expression of the gene at a higher level than occurs in the corresponding nontransfected cell or allows a pattern of regulation or induction that is different from the corresponding nontransfected cell.

Immortalized cells which produce a therapeutic protein produced by the methods described herein can be used in gene therapy. The immortalized cells can be enclosed in one of a number of semipermeable barrier devices. The permeability properties of the device are such that the cells are prevented from leaving the device upon implantation into an animal, but the therapeutic.product is freely permeable and can leave the barrier device and enter the local space surrounding the implant or enter the systemic circulation. A number of filtration membranes can be used for this purpose, including, but not limited to, cellulose, cellulose acetate, nitrocellulose, polysulfone, polyvinylidene difluoride, polyvinyl chloride polymers and polymers of polyvinyl chloride derivatives. Alternatively, barrier devices can be utilized to allow primary, secondary, or immortalized cells from another species to be used for gene therapy in humans. The use of cells from other species can be desirable in cases where the non-human cells are advantageous for protein production purposes or in cases where the non-human protein is therapeutically useful, for example, the use of cells derived from salmon for the production of salmon calcitonin and the use of cells derived from pigs for the production of porcine insulin.

Cells from non-human species can also be used for in vitro protein production. These cells can be immortalized, primary, or secondary cells which produce a therapeutic protein produced by the methods described here and in the U.S. patent applications described herein. The use of cells from other species may be desirable in cases where the non-human cells are advantageous for protein production purposes (for example CHO cells) or in cases where the non-human protein is therapeutically or commercially useful, for example, the use of cells derived from salmon for the production of salmon calcitonin, the use of cells derived from pigs for the production of porcine insulin, and the use of bovine cells for the production of bovine growth hormone.

Transfected cells of the present invention are useful in a number of applications in humans and animals. In one embodiment, the cells can be implanted into a human or an animal for protein delivery in the human or animal. For example, human growth hormone (hGH), human EPO (hEPO), or human insulinotropin can be delivered systemically in humans for therapeutic benefits. Barrier devices, through which the therapeutic product is freely permeable, can be used to retain cells in a fixed position in vivo or to protect and isolate the cells from the host's immune system. Barrier devices are particularly useful and allow transfected immortalized cells, transfected cells from another species (transfected xenogeneic cells), or cells from a nonhistocompatibility-matched donor (transfected allogeneic cells) to be implanted for treatment of human or animal conditions or for agricultural uses (i.e., meat and dairy production). Barrier devices also allow convenient short-term (i.e., transient) therapy by providing ready access to the cells for removal when the treatment regimen is to be halted for any reason.

Transfected cells of the present invention are also useful for eliciting antibody production or for immunizing humans and animals against pathogenic agents. Implanted transfected cells can be used to deliver immunizing antigens that result in stimulation of the host's cellular and humoral immune responses. These immune responses can be designed for protection of the host from future infectious agents (i.e., for vaccination), to stimulate and augment the disease-fighting capabilities directed against an ongoing infection, or to produce antibodies directed against the antigen produced in vivo by the transfected cells that can be useful for therapeutic or diagnostic purposes. Removable barrier devices can be used to allow a simple means of terminating exposure to the antigen. Alternatively, the use of cells that will ultimately be rejected (xenogeneic or allogeneic transfected cells) can be used to limit exposure to the antigen since antigen production will cease when the cells have been rejected.

### Explanation of the Examples

As described herein, Applicants have demonstrated that DNA can be introduced into primary, secondary or immortalized vertebrate cells and integrated into the genome of the transfected primary or secondary cells by homologous recombination. That is, they have demonstrated gene targeting in primary, secondary and immortalized mammalian cells. They have further demonstrated that the exogenous DNA has the desired function in the homologously recombinant (HR) cells and that correctly targeted cells can be identified on the basis of a detectable phenotype conferred by the properly targeted DNA.

In addition, the present invention allows a method of protein production using transfected primary, secondary or immortalized cells. Examples 1g, lj, 2, 3 and 4 describe protein production by targeting and activation of a selected endogenous gene.

The applicants also describe DNA constructs and methods for amplifying an endogenous cellular gene that has been activated by gene targeting (Examples 1f-1k and Example 3) and further describe methods by which a gene can be inserted at a preselected site in the genome of a primary, secondary, or immortalized cell by gene targeting (Example 1d).

Applicants describe construction of a plasmid useful for targeting to a particular locus (the HPRT locus) in the human genome and selection based upon a drug resistant phenotype (Example la). This plasmid is designated pE3Neo and its integration into the cellular genomes at the HPRT locus produces cells which have an hprt·, 6-TG resistant phenotype and are also G418 resistant. As described, they have shown that pE3Neo functions properly in gene targeting in an established human fibroblast cell line (Example 1b), by demonstrating localization of the DNA introduced into established cells within exon 3 of the HPRT gene.

In addition, Applicants demonstrate gene targeting in primary and secondary human skin fibroblasts using pE3Neo (Example 1c). The subject application further demonstrates that modification of DNA termini enhances targeting of DNA into genomic DNA (Examples 1c and le).

Examples if-lh and 2 illustrate embodiments in which the normal regulatory sequences upstream of the human EPO gene are altered to allow expression of hEPO in primary or secondary fibroblast strains which do not express EPO in detectable quantities in their untransfected state. In one embodiment the product of targeting leaves the normal EPO protein intact, but under the control of the mouse metallothionein promoter. Examples 1i and 1j demonstrate the use of similar targeting constructs to activate the endogenous growth hormone gene in primary or secondary human fibroblasts. In other embodiments described for activating EPO expression in human fibroblasts, the products of targeting events are chimeric transcription units, in which the first exon of the human growth hormone gene is positioned upstream of EPO exons 2-5. The product of transcription (controlled by the mouse metallothionein promoter), splicing, and translation is a protein in which amino acids 1-4 of the hEPO signal peptide are replaced with amino acid residues 1-3 of hGH. The chimeric portion of this protein, the signal peptide, is removed prior to secretion from cells. Example 5 describes construction of a targeting vector, designated pREP04 for dual selection and selection of cells in which the dhfr gene is amplified. Plasmid pREP04 has been used to amplify the human EPO (hEPO) locus in HT1080 cells (an immortalized human cell line) after activation of the endogenous hEPO gene by homologous recombination. As described, stepwise selection in methotrexate-containing media resulted in a 70-fold increase in hEPO production in cells resistant to 0.4 µM methotrexate.

The Examples provide methods for activating or for activating and amplifying endogenous genes by gene targeting which do not require manipulation or other uses of the target genes' protein coding regions. By these methods, normally inactive genes can be activated in cells that have properties desirable for in vitro protein production (e.g., pharmaceutics) or in vivo protein delivery methods (e.g. gene therapy). Figures S and 6 illustrate two strategies for transcriptionally activating the hEPO gene.

Using the methods and DNA constructs or plasmids taught herein or modifications thereof which are apparent to one of ordinary skill in the art, exogenous DNA which encodes a therapeutic product (e.g., protein, ribozyme, nucleic acid) can be inserted at preselected sites in the genome of vertebrate (e.g., mammalian, both human and nonhuman) primary or secondary cells.

The present invention will now be illustrated by the following examples, which are not intended to be limiting in any way. (If certain examples do not include all of the features of the claims they are are retained for information purposes.)

### EXAMPLES

### EXAMPLE 1. PRODUCTION OF TRANSFECTED CELL STRAINS BY GENE TARGETING

Gene targeting occurs when transfecting DNA either integrates into or partially replaces chromosomal DNA sequences through a homologous recombinant event. While such events can occur in the course of any given transfection experiment, they are usually masked by a vast excess of events in which plasmid DNA integrates by nonhomologous, or illegitimate, recombination.

### a. GENERATION OF A CONSTRUCT USEFUL FOR SELECTION OF GENE TARGETING EVENTS IN HUMAN CELLS

One approach to selecting the targeted events is by genetic selection for the loss of a gene function due to the integration of transfecting DNA. The human HPRT locus encodes the enzyme hypoxanthine-phosphoribosyl transferase. hprt· cells can be selected for by growth in medium containing the nucleoside analog 6-thioguanine (6-TG): cells with the wild-type (HPRT+) allele are killed by 6-TG, while cells with mutant (hprt⁻) alleles can survive. Cells harboring targeted events which disrupt HPRT gene function are therefore selectable in 6-TG medium.

To construct a plasmid for targeting to the HPRT locus, the 6.9 kb HindIII fragment extending from positions 11,960-18,869 in the HPRT sequence (Genebank name HUMHPRTB; Edwards, A. et al., Genomics 6:593-608 (1990)) and including exons 2 and 3 of the HPRT gene, is subcloned into the HindIII site of pUC12. The resulting clone is cleaved at the unique XhoI site in exon 3 of the HPRT gene fragment and the 1.1 kb SalI-XhoI fragment containing the neo gene from pMClNeo (Stratagene) is inserted, disrupting the coding sequence of exon 3. One orientation, with the direction of neo transcription opposite that of HPRT transcription was chosen and designated pE3Neo. The replacement of the normal HPRT exon 3 with the neo-disrupted version will result in an hprt⁻, 6-TG resistant phenotype. Such cells will also be G418 resistant.

### b. GENE TARGETING IN AN ESTABLISHED HUMAN FIBROBLAST CELL LINE

As a demonstration of targeting in immortalized cell lines, and to establish that pE3Neo functions properly in gene targeting, the human fibrosarcoma cell line HT1080 (ATCC CCL 121) was transfected with pE3Neo by electroporation.

HT1080 cells were maintained in HAT (hypoxanthine/ aminopterin/xanthine) supplemented DMEM with 15% calf serum (Hyclone) prior to electroporation. Two days before electroporation, the cells are switched to the same medium without aminopterin. Exponentially growing cells were trypsinized and diluted in DMEM/15% calf serum, centrifuged, and resuspended in PBS (phosphate buffered saline) at a final cell volume of 13.3 million cells per ml. pE3Neo is digested with HindIII, separating the 8 kb HPRT-neo fragment from the pUC12 backbone, purified by phenol extraction and ethanol precipitation, and resuspended at a concentration of 600 µg/ml. 50 µl (30 µg) was added to the electroporation cuvette (0.4 cm electrode gap; Bio-Rad Laboratories), along with 750 µl of the cell suspension (10 million cells). Electroporation was at 450 volts, 250 µFarads (Bio-Rad Gene Pulser; Bio-Rad Laboratories). The contents of the cuvette were immediately added to DMEM with 15% calf serum to yield a cell suspension of 1 million cells per 25 ml media. 25 ml of the treated cell suspension was plated onto 150 mm diameter tissue culture dishes and incubated at 37°C, 5% CO₂. 24 hrs later, a G418 solution was added directly to the plates to yield a final concentration of 800 µg/ml G418. Five days later the media was replaced with DMEM/15% calf serum/800 µg/ml G418. Nine days after electroporation, the media was replaced with DMEM/15% calf serum/800 µg/ml G418 and 10 µM 6-thioguanine. Colonies resistant to G418 and 6-TG were picked using cloning cylinders 14-16 days after the dual selection was initiated.

The results of five representative targeting experiments in HT1080 cells are shown in Table 1.

**TABLE 1**

| Transfection | Number of Treated Cells | Number of G418^{r} 6-TG^{r} Clones |
|---|---|---|
| 1 | 1 x 10⁷ | 32 |
| 2 | 1 x 10⁷ | 28 |
| 3 | 1 x 10⁷ | 24 |
| 4 | 1 x 10⁷ | 32 |
| 5 | 1 x 10⁷ | 66 |

For transfection 5, control plates designed to determine the overall yield of G418^{r} colonies indicated that 33,700 G418^{r} colonies could be generated from the initial 1 x 10⁷ treated cells. Thus, the ratio of targeted to non-targeted events is 66/33,700, or 1 to 510. In the five experiments combined, targeted events arise at a frequency of 3.6 x 10⁶, or 0.00036% of treated cells.

Restriction enzyme and Southern hybridization experiments using probes derived from the neo and HPRT genes localized the neo gene to the HPRT locus at the predicted site within HPRT exon 3.

### c. GENE TARGETING IN PRIMARY AND SECONDARY HUMAN SKIN FIBROBLASTS

pE3Neo is digested with HindIII, separating the 8 kb HPRT-neo fragment from the pUC12 backbone, and purified by phenol extraction and ethanol precipitation. DNA was resuspended at 2 mg/ml. Three million secondary human foreskin fibroblasts cells in a volume of 0.5 ml were electroporated at 250 volts and 960 µFarads, with 100 µg of HindIII pE3Neo (50 µl). Three separate transfections were performed, for a total of 9 million treated cells. Cells are processed and selected for G418 resistance. 500,000 cells per 150 mm culture dish were plated for G418 selection. After 10 days under selection, the culture medium is replaced with human fibroblast nutrient medium containing 400 µg/ml G418 and 10 µM 6-TG. Selection with the two drug combination is continued for 10 additional days. Plates are scanned microscopically to localize human fibroblast colonies resistant to both drugs. The fraction of G418^{r} t-TG^{r} colonies is 4 per 9 million treated cells. These colonies constitute 0.0001% (or 1 in a million) of all cells capable of forming colonies. Control plates designed to determine the overall yield of G418^{r} colonies indicated that 2,850 G418^{r} colonies could be generated from the initial 9 x 10⁶ treated cells. Thus, the ratio of targeted to non-targeted events is 4/2,850, or 1 to 712. Restriction enzyme and Southern hybridization experiments using probes derived from the neo and HPRT genes were used to localize the neo gene to the HPRT locus at the predicted site within HPRT exon 3 and demonstrate that targeting had occurred in these four clonal cell strains. Colonies resistant to both drugs have also been isolated by transfecting primary cells (1/3.0 x 10⁷).

The results of several pE3Neo targeting experiments are summarized in Table 2. HindIII digested pE3Neo was either transfected directly or treated with exonuclease III to generate 5' single-stranded overhangs prior to transfection (see Example 1c). DNA preparations with single-stranded regions ranging from 175 to 930 base pairs in length were tested. Using pE3neo digested with HindIII alone, 1/799 G418-resistant colonies were identified by restriction enzyme and Southern hybridization analysis as having a targeted insertion of the neo gene at the HPRT locus (a total of 24 targeted clones were isolated). Targeting was maximally stimulated (approximately 10-fold stimulation) when overhangs of 175 bp were used, with 1/80 G418^{r} colonies displaying restriction fragments that are diagnostic for targeting at HPRT (a total of 9 targeted clones were isolated). Thus, using the conditions and recombinant DNA constructs described here, targeting is readily observed in normal human fibroblasts and the overall targeting frequency (the number of targeted clones divided by the total number of clones stably transfected to G418-resistance) can be stimulated by transfection with targeting constructs containing single-stranded overhanging tails, by the method as described in Example 1e.

**TABLE 2**

| TARGETING TO THE HPRT LOCUS IN HUMAN FIBROBLASTS | | | |
|---|---|---|---|
| pE3neo Treatment | Number of Experiments | Number Targeted Per G418^{r} Colony | Total Number of Targeted Clone |
| HindIII digest | 6 | 1/799 | 24 |
| 175 bp overhang | 1 | 1/80 | 9 |
| 350 bp overhang | 3 | 1/117 | 20 |
| 930 bp overhang | 1 | 1/144 | 1 |

### d. GENERATION OF A CONSTRUCT FOR TARGETED INSERTION OF A GENE OF THERAPEUTIC INTEREST INTO THE HUMAN GENOME AND ITS USE IN GENE TARGETING

A variant of pE3Neo, in which a gene of therapeutic interest is inserted within the HPRT coding region, adjacent to or near the neo gene, can be used to target a gene of therapeutic interest to a specific position in a recipient primary or secondary cell genome. Such a variant of pE3Neo can be constructed for targeting the hGH gene to the HPRT locus.

pXGH5 (schematically presented in Figure 1) is digested with EcoRI and the 4.1 kb fragment containing the hGH gene and linked mouse metallothionein (mMT) promoter is isolated. The EcoRI overhangs are filled in with the Klenow fragment from E. coli DNA polymerase. Separately, pE3Neo is digested with XhoI, which cuts at the junction of the neo fragment and HPRT exon 3 (the 3' junction of the insertion into exon 3). The XhoI overhanging ends of the linearized plasmid are filled in with the Klenow fragment from E. coli DNA polymerase, and the resulting fragment is ligated to the 4.1 kb blunt-ended hGH-mMT fragment. Bacterial colonies derived from the ligation mixture are screened by restriction enzyme analysis for a single copy insertion of the hGH-mMT fragment and one orientation, the hGH gene transcribed in the same direction as the neo gene, is chosen and designated pE3Neo/hGH. pE3Neo/hGH is digested with HindIII, releasing the 12.1 kb fragment containing HPRT, neo and mMT-hGH sequences. Digested DNA is treated and transfected into primary or secondary human fibroblasts as described in Example 1c. G418^{r} TG^{r} colonies are selected and analyzed for targeted insertion of the mMT-hGH and neo sequences into the HPRT gene as described in Example 1c. Individual colonies are assayed for hGH expression using a commercially available immunoassay (Nichols Institute).

Secondary human fibroblasts were transfected with pE3Neo/hGH and thioguanine-resistant colonies were analyzed for stable hGH expression and by restriction enzyme and Southern hybridization analysis. Of thirteen TG^{r} colonies analyzed, eight colonies were identified with an insertion of the hGH gene into the endogenous HPRT locus. All eight strains stably expressed significant quantities of hGH, with an average expression level of 22.7 µg/10⁶ cells/24 hours. Alternatively, plasmid pE3neoEPO, Figure 4, may be used to target EPO to the human HPRT locus.

The use of homologous recombination to target a gene of therapeutic interest to a specific position in a cell's genomic DNA can be expanded upon and made more useful for producing products for therapeutic purposes (e.g., pharmaceutics, gene therapy) by the insertion of a gene through which cells containing amplified copies of the gene can be selected for by exposure of the cells to an appropriate drug selection regimen. For example, pE3neo/hGH (Example ld) can be modified by inserting the dhfr, ada, or CAD gene at a position immediately adjacent to the hGH or neo genes in pE3neo/hGH. Primary, secondary, or immortalized cells are transfected with such a plasmid and correctly targeted events are identified. These cells are further treated with increasing concentrations of drugs appropriate for the selection of cells containing amplified genes (for dhfr, the selective agent is methotrexate, for CAD the selective agent is N-(phosphonacetyl)-L-aspartate (PALA), and for ada the selective agent is an adenine nucleoside (e.g., alanosine). In this manner the integration of the gene of therapeutic interest will be coamplified along with the gene for which amplified copies are selected. Thus, the genetic engineering of cells to produce genes for therapeutic uses can be readily controlled by preselecting the site at which the targeting construct integrates and at which the amplified copies reside in the amplified cells.

### e. MODIFICATION OF DNA TERMINI TO ENHANCE TARGETING

Several lines of evidence suggest that 3'-overhanging ends are involved in certain homologous recombination pathways of E. coli, bacteriophage, S. cerevisiae and Xenopus laevis. In Xenopus laevis oocytes, molecules with 3'-overhanging ends of several hundred base pairs in length underwent recombination with similarly treated molecules much more rapidly after microinjection than molecules with very short overhangs (4 bp) generated by restriction enzyme digestion. In yeast, the generation of 3'-overhanging ends several hundred base pairs in length appears to be a rate limiting step in meiotic recombination. No evidence for an involvement of 3'-overhanging ends in recombination in human cells has been reported, and in no case have modified DNA substrates of any sort been shown to promote targeting (one form of homologous recombination) in any species. In human cells, the effect of 3'-overhanging ends on targeting is untested. The experiment described in the following example and Example 1c suggests that 5'-overhanging ends are effective for stimulating targeting in primary, secondary and immortalized human fibroblasts.

There have been no reports on the enhancement of targeting by modifying the ends of the transfecting DNA molecules. This example serves to illustrate that modification of the ends of linear DNA molecules, by conversion of the molecules' termini from a double-stranded form to a single-stranded form, can stimulate targeting into the genome of primary and secondary human fibroblasts.

1100 µg of plasmid pE3Neo (Example la) is digested with HindIII. This DNA can be used directly after phenol extraction and ethanol precipitation, or the 8 kb HindIII fragment containing only HPRT and the neo gene can be separated away from the pUC12 vector sequences by gel electrophoresis. ExoIII digestion of the HindIII digested DNA results in extensive exonucleolytic digestion at each end, initiating at each free 3' end, and leaving 5'-overhanging ends. The extent of exonucleolytic action and, hence, the length of the resulting 5'-overhangs, can be controlled by varying the time of ExoIII digestion. ExoIII digestion of 100 µg of HindIII digested pE3Neo is carried out according to the supplier's recommended conditions, for times of 30 sec, 1 min, 1.5 min, 2 min, 2.5 min, 3 min, 3.5 min, 4 min, 4.5 min, and 5 min. To monitor the extent of digestion an aliquot from each time point, containing 1 µg of ExoIII treated DNA, is treated with mung bean nuclease (Promega), under conditions recommended by the supplier, and the samples fractionated by gel electrophoresis. The difference in size between non-treated, HindIII digested pE3Neo and the same molecules treated with ExoIII and mung bean nuclease is measured. This size difference divided by two gives the average length of the 5'-overhang at each end of the molecule. Using the time points described above and digestion at 30°, the 5'-overhangs produced should range from 100 to 1,000 bases.

60 µg of ExoIII treated DNA (total HindIII digest of pE3Neo) from each time point is purified and electroporated into primary, secondary, or immortalized human fibroblasts under the conditions described in Example 1c. The degree to which targeting is enhanced by each ExoIII treated preparation is quantified by counting the number of G418^{r} 6-TG^{r} colonies and comparing these numbers to targeting with HindIII digested pE3Neo that was not treated with ExoIII.

The effect of 3'-overhanging ends can also be quantified using an analogous system. In this case HindIII digested pE3Neo is treated with bacteriophage T7 gene 6 exonuclease (United States Biochemicals) for varying time intervals under the supplier's recommended conditions. Determination of the extent of digestion (average length of 3'-overhang produced per end) and electroporation conditions are as described for ExoIII treated DNA. The degree to which targeting is enhanced by each T7 gene 6 exonuclease treated preparation is quantified by counting the number of G418^{r} 6-TG^{r} colonies and comparing these numbers to targeting with HindIII digested pE3Neo that was not treated with T7 gene 6 exonuclease.

Other methods for generating 5' and 3' overhanging ends are possible, for example, denaturation and annealing of two linear molecules that partially overlap with each other will generate a mixture of molecules, each molecule having 3'-overhangs at both ends or 5'-overhangs at both ends, as well as reannealed fragments indistinguishable from the starting linear molecules. The length of the overhangs is determined by the length of DNA that is not in common between the two DNA fragments.

### f. CONSTRUCTION OF TARGETING PLASMIDS FOR PLACING THE HUMAN ERYTHROPOIETIN GENE UNDER THE CONTROL OF THE MOUSE METALLOTHIONEIN PROMOTER IN PRIMARY, SECONDARY AND IMMORTALIZED HUMAN FIBROBLASTS

The following serves to illustrate one embodiment of the present invention, in which the normal positive and negative regulatory sequences upstream of the human erythropoietin (EPO) gene are altered to allow expression of human erythropoietin in primary, secondary or immortalized human fibroblasts, which do not express EPO in significant quantities as obtained.

A region lying exclusively upstream of the human EPO coding region can be amplified by PCR. Three sets of primers useful for this purpose were designed after analysis of the published human EPO [Genbank designation HUMERPA; Lin, F-K., et al., Proc. Natl. Acad. Sci., USA 82:7580-7584 (1985)]. These primer pairs can amplify fragments of 609, 603, or 590 bp.

**TABLE 3**

| Primer | HUMERPA Coordinate | Sequence | Fragment Size |
|---|---|---|---|
| F1 | 2 - 20 | 5' AGCTTCTGGGCTTCCAGAC (SEQ ID NO 1) | |
| R2 | 610 - 595 | 5' GGGGTCCCTCAGCGAC (SEQ ID NO 2) | 609 bp |
| F2 | 8 - 24 | 5' TGGGCTTCCAGACCCAG (SEQ ID NO 3) | |
| R2 | 610 - 595 | 5' GGGGTCCCTCAGCGAC | 603 bp |
| F3 | 21 - 40 | 5' CCAGCTACTTTGCGGAACTC (SEQ ID NO 4) | |
| R2 | 610 - 595 | 5' GGGGTCCCTCAGCGAC | 590 bp |

The three fragments overlap substantially and are interchangeable for the present purposes. The 609 bp fragment, extending from -623 to -14 relative to the translation start site (HUMERPA nucleotide positions 2 to 610), is ligated at both ends with ClaI linkers. The resulting ClaI-linked fragment is digested with ClaI and inserted into the ClaI site of pBluescriptIISK/+ (Stratagene), with the orientation such that HUMERPA nucleotide position 610 is adjacent to the SalI site in the plasmid polylinker). This plasmid, p5'EPO, can be cleaved, separately, at the unique FspI or SfiI sites in the EPO upstream fragment (HUMERPA nucleotide positions 150 and 405, respectively) and ligated to the mouse metallothionein promoter. Typically, the 1.8 kb EcoRI-BglII from the mMT-I gene [containing no mMT coding sequences; Hamer, D.H. and Walling M., J. Mol. Appl. Gen. 1:273 288 (1982); this fragment can also be isolated by known methods from mouse genomic DNA using PCR primers designed from analysis of mMT sequences available from Genbank; i.e., MUSMTI, MUSMTIP, MUSMTIPRM] is made blunt- ended by known methods and ligated with SfiI digested (also made blunt-ended) or FspI digested p5'EPO. The orientations of resulting clones are analyzed and those in which the former mMT BglII site is proximal to the SalI site in the plasmid polylinker are used for targeting primary and secondary human fibroblasts. This orientation directs mMT transcription towards HUMERPA nucleotide position 610 in the final construct. The resulting plasmids are designated p5'EPO-mMTF and p5'EPO-mMTS for the mMT insertions in the FspI and SfiI sites, respectively.

Additional upstream sequences are useful in cases where it is desirable to modify, delete and/or replace negative regulatory elements or enhancers that lie upstream of the initial target sequence. In the case of EPO, a negative regulatory element that inhibits EPO expression in extrahepatic and extrarenal tissues [Semenza, G.L. et al., Mol. Cell. Biol. 10:930-938 (1990)] can be deleted. A series of deletions within the 6 kb fragment are prepared. The deleted regions can be replaced with an enhancer with broad host-cell activity [e.g. an enhancer from the Cytomegalovirus (CMV)].

The orientation of the 609 bp 5'EPO fragment in the pBluescriptIISK/+ vector was chosen since the HUMERPA sequences are preceded on their 5' end by a BamHI (distal) and HindIII site (proximal). Thus, a 6 kb BamHI-HindIII fragment normally lying upstream of the 609 bp fragment [Semenza, G. L. et al., Mol. Cell. Biol. 10:930-938 (1990)] can be isolated from genomic DNA by known methods. For example, a bacteriophage, cosmid, or yeast artificial chromosome library could be screened with the 609 bp PCR amplified fragment as a probe. The desired clone will have a 6 kb BamHI-HindIII fragment and its identity can be confirmed by comparing its restriction map from a restriction map around the human EPO gene determined by known methods. Alternatively, constructing a restriction map of the human genome upstream of the EPO gene using the 609 bp fragment as a probe can identify enzymes which generate a fragment originating between HUMERPA coordinates 2 and 609 and extending past the upstream BamHI site; this fragment can be isolated by gel electrophoresis from the appropriate digest of human genomic DNA and ligated into a bacterial or yeast cloning vector. The correct clone will hybridize to the 609 bp 5'EPO probe and contain a 6 kb BamHI-HindIII fragment. The isolated 6 kb fragment is inserted in the proper orientation into p5'EPO, p5'EPO-mM-TF, or p5'EPO-mMTS (such that the HindIII site is adjacent to HUMERPA nucleotide position 2). Additional upstream sequences can be isolated by known methods, using chromosome walking techniques or by isolation of yeast artificial chromosomes hybridizing to the 609 bp 5'EPO probe.

The cloning strategies described above allow sequences upstream of EPO to be modified in vitro for subsequent targeted transfection of primary, secondary or immortalized human fibroblasts. The strategies describe simple insertions of the mMT promoter, as well as deletion of the negative regulatory region, and deletion of the negative regulatory region and replacement with an enhancer with broad host-cell activity.

### g. TARGETING TO SEQUENCES FLANKING THE HUMAN EPO GENE AND ISOLATION OF TARGETED PRIMARY. SECONDARY AND IMMORTALIZED HUMAN FIBROBLASTS BY SCREENING

For targeting, the plasmids are cut with restriction enzymes that free the insert away from the plasmid backbone. In the case of p5'EPO-mMTS, HindIII and SaII digestion releases a targeting fragment of 2.4 kb, comprised of the 1.8 kb mMT promoter flanked on the 5' and 3' sides by 405 bp and 204 base pairs, respectively, of DNA for targeting this construct to the regulatory region of the EPO gene. This DNA or the 2.4 kb targeting fragment alone is purified by phenol extraction and ethanol precipitation and transfected into primary or secondary human fibroblasts under the conditions described in Example 1c. Transfected cells are plated onto 150 mm dishes in human fibroblast nutrient medium. 48 hours later the cells are plated into 24 well dishes at a density of 10,000 cells/cm² [approximately 20,000 cells per well; if targeting occurs at a rate of 1 event per 10⁶ clonable cells (Example lc, then about 50 wells would need to be assayed to isolate a single expressing colony]. Cells in which the transfecting DNA has targeted to the homologous region upstream of EPO will express EPO under the control of the mMT promoter. After 10 days, whole well supernatants are assayed for EPO expression using a commercially available immunoassay kit (Amgen). Clones from wells displaying EPO synthesis are isolated using known methods, typically by assaying fractions of the heterogenous populations of cells separated into individual wells or plates, assaying fractions of these positive wells, and repeating as needed, ultimately isolating the targeted colony by screening 96-well microtiter plates seeded at one cell per well. DNA from entire plate lysates can also be analyzed by PCR for amplification of a fragment using a mMT specific primer in conjunction with a primer lying upstream of HUMERPA nucleotide position 1. This primer pair should amplify a DNA fragment of a size precisely predicted based on the DNA sequence. Positive plates are trypsinized and replated at successively lower dilutions, and the DNA preparation and PCR steps repeated as needed to isolate targeted cells.

The targeting schemes herein described can also be used to activate hGH expression in immortalized human cells (for example, HT1080 (ATCC CCL 121) fibroblasts, HeLa (ATCC CCL 2) cells, MCF-7 (ATCC HTB 22) breast cancer cells, K-562 (ATCC CCL 243) leukemia cells, KB (ATCC CCL 17) carcinoma cells or 2780AD (Van der Bliek, A.M. et al., Cancer Res. 48:5927-5932 (1988)) ovarian carcinoma cells) for the purposes of producing hGH for conventional pharmaceutic delivery.

### h. TARGETING TO SEQUENCES FLANKING THE HUMAN EPO GENE AND ISOLATION OF TARGETED PRIMARY, SECONDARY AND IMMORTALIZED HUMAN FIBROBLASTS BY A POSITIVE OR A COMBINED POSITIVE/NEGATIVE SELECTION SYSTEM

The strategy for constructing p5'EPO-mMTF, p5'EPO-mM-TS, and derivatives of such with the additional upstream 6 kb BamHI-HindIII fragment can be followed with the additional step of inserting the neo gene adjacent to the mMT promoter. In addition, a negative selection marker, for example, gpt [from pMSG (Pharmacia) or another suitable source], can be inserted adjacent to the HUMERPA sequences in the pBluescriptIISK/+ polylinker. In the former case, G418^{r} colonies are isolated and screened by PCR amplification or restriction enzyme and Southern hybridization analysis of DNA prepared from pools of colonies to identify targeted colonies. In the latter case, G418^{r} colonies are placed in medium containing 6-thioxanthine to select against the integration of the gpt gene [Besnard, C. et al., Mol. Cell. Biol. 7:4139-4141 (1987)]. In addition, the HSV-TK gene can be placed on the opposite side of the insert as gpt, allowing selection for neo and against both gpt and TK by growing cells in human fibroblast nutrient medium containing 400 µg/ml G418, 100 µM 6-thioxanthine, and 25 µg/ml gancyclovir. The double negative selection should provide a nearly absolute selection for true targeted events and Southern blot analysis provides an ultimate confirmation.

The targeting schemes herein described can also be used to activate hEPO expression in immortalized human cells (for example, HT1080 fibroblasts, HeLa cells, MCF-7 breast cancer cells, K-562 leukemia cells, KB carcinoma cells or 2780AD ovarian carcinoma cells) for the purposes of producing hEPO for conventional pharmaceutic delivery.

### i. CONSTRUCTION OF TARGETING PLASMIDS FOR PLACING THE HUMAN GROWTH HORMONE GENE UNDER THE CONTROL OF THE MOUSE METALLOTHIONEIN PROMOTER IN PRIMARY, SECONDARY OR IMMORTALIZED HUMAN FIBROBLASTS

The following example serves to illustrate one embodiment of the present invention, in which the normal regulatory sequences upstream of the human growth hormone gene are altered to allow expression of human growth hormone in primary, secondary or immortalized human fibroblasts.

Targeting molecules similar to those described in Example 1f for targeting to the EPO gene regulatory region are generated using cloned DNA fragments derived from the 5' end of the human growth hormone N gene. An approximately 1.8 kb fragment spanning HUMGHCSA (Genbank Entry) nucleotide positions 3787-5432 (the positions of two EcoNI sites which generate a convenient sized fragment for cloning or for diagnostic digestion of subclones involving this fragment) is amplified by PCR primers designed by analysis of the HUMGHCSA sequence in this region. This region extends from the middle of hGH gene N intron 1 to an upstream position approximately 1.4 kb 5' to the translational start site. pUC12 is digested with EcoRI and BamHI, treated with Klenow to generate blunt ends, and recircularized under dilute conditions, resulting in plasmids which have lost the EcoRI and BamHI sites. This plasmid is designated pUC12XEB. HindIII linkers are ligated onto the amplified hGH fragment and the resulting fragment is digested with HindIII and ligated to HindIII digested pUC12XEB. The resulting plasmid, pUC12XEB-5'hGH, is digested with EcoRI and BamHI, to remove a 0.5 kb fragment lying immediately upstream of the hGH transcriptional initiation site. The digested DNA is ligated to the 1.8 kb EcoRI-BglII from the mMT-I gene [containing no mMT coding sequences; Hamer, D.H. and Walling, M., J. Mol. Appl. Gen. 1:273-288 (1982); the fragment can also be isolated by known methods from mouse genomic DNA using PCR primers designed from analysis of mMT sequences available from Genbank; i.e., MUSMTI, MUSMTIP, MUSMTIPRM]. This plasmid p5'hGH-mMT has the mMT promoter flanked on both sides by upstream hGH sequences.

The cloning strategies described above allow sequences upstream of hGH to be modified in vitro for subsequent targeted transfection of primary, secondary or immortalized human fibroblasts. The strategy described a simple insertion of the mMT promoter. Other strategies can be envisioned, for example, in which an enhancer with broad host-cell specificity is inserted upstream of the inserted mMT sequence.

### j. TARGETING TO SEQUENCES FLANKING THE HUMAN hGH GENE AND ISOLATION OF TARGETED PRIMARY, SECONDARY AND IMMORTALIZED HUMAN FIBROBLASTS BY SCREENING

For targeting, the plasmids are cut with restriction enzymes that free the insert away from the plasmid backbone. In the case of p5'hGH-mMT, HindIII digestion releases a targeting fragment of 2.9 kb, comprised of the 1.8 kb mMT promoter flanked on the 5' end 3' sides by DNA for targeting this construct to the regulatory region of the hGH gene. This DNA or the 2.9 kb targeting fragment alone is purified by phenol extraction and ethanol precipitation and transfected into primary or secondary human fibroblasts under the conditions described in Example 11. Transfected cells are plated onto 150 mm dishes in human fibroblast nutrient medium. 48 hours later the cells are plated into 24 well dishes at a density of 10,000 cells/cm² [approximately 20,000 cells per well; if targeting occurs at a rate of 1 event per 10⁶ clonable cells (Example 1c), then about 50 wells would need to be assayed to isolate a single expressing colony]. Cells in which the transfecting DNA has targeted to the homologous region upstream of hGH will express hGH under the control of the mMT promoter. After 10 days, whole well supernatants are assayed for hGH expression using a commercially available immunoassay kit (Nichols). Clones from wells displaying hGH synthesis are isolated using known methods, typically by assaying fractions of the heterogenous populations of cells separated into individual wells or plates, assaying fractions of these positive wells, and repeating as needed, ultimately isolated the targeted colony by screening 96-well microtiter plates seeded at one cell per well. DNA from entire plate lysates can also be analyzed by PCR for amplification of a fragment using a mMT specific primer in conjunction with a primer lying downstream of HUMGHCSA nucleotide position 5,432. This primer pair should amplify a DNA fragment of a size precisely predicted based on the DNA sequence. Positive plates are trypsinized and replated at successively lower dilutions, and the DNA preparation and PCR steps repeated as needed to isolate targeted cells.

The targeting schemes herein described can also be used to activate hGH expression in immortalized human cells (for example, HT1080 fibroblasts, HeLa cells, MCF-7 breast cancer cells, K-562 leukemia cells, KB carcinoma cells or 2780AD ovarian carcinoma cells) for the purposes of producing hGH for conventional pharmaceutic delivery.

### k. TARGETING TO SEQUENCES FLANKING THE HUMAN hGH GENE AND ISOLATION OF TARGETED PRIMARY SECONDARY AND IMMORTALIZE HUMAN FIBROBLASTS BY A POSITIVE OR A COMBINED POSITIVE/NEGATIVE SELECTION SYSTEM

The strategy for constructing p5'hGH-mMT can be followed with the additional step of inserting the neo gene adjacent to the mMT promoter. In addition, a negative selection marker, for example, gpt [from pMSG (Pharmacia) or another suitable source], can be inserted adjacent to the HUMGHCSA sequences in the pUC12 poly-linker. In the former case, G418^{r} colonies are isolated and screened by PCR amplification or restriction enzyme and Southern hybridization analysis of DNA prepared from pools of colonies to identify targeted colonies. In the latter case, G418^{r} colonies are placed in medium containing thioxanthine to select against the integration of the gpt gene (Besnard, C. et al., Mol. Cell. Biol. 7: 4139-4141 (1987)]. In addition, the HSV-TK gene can be placed on the opposite side of the insert as gpt, allowing selection for neo and against both gpt and TK by growing cells in human fibroblast nutrient medium containing 400 µg/ml G418, 100 µM 6-thioxanthine, and 25 µg/ml gancyclovir. The double negative selection should provide a nearly absolute selection for true targeted events. Southern hybridization analysis is confirmatory.

The targeting schemes herein described can also be used to activate hGH expression in immortalized human cells (for example, HT1080 fibroblasts, HeLa cells, MCF-7 breast cancer cells, K-562 leukemia cells, KB carcinoma cells or 2780AD ovarian carcinoma cells) for the purposes of producing hGH for conventional pharmaceutic delivery.

The targeting constructs described in Examples 1f and 1i, and used in Examples 1g, 1h, 1j and 1k can be modified to include an amplifiable selectable marker (e.g., ada, dhfr, or CAD) which is useful for selecting cells in which the activated endogenous gene, and the amplifiable selectable marker, are amplified. Such cells, expressing or capable of expressing the endogenous gene encoding a therapeutic product can be used to produce proteins (e.g., hGH and hEPO) for conventional pharmaceutic delivery or for gene therapy.

### 1. TRANSFECTION OF PRIMARY AND SECONDARY FIBROBLASTS WITH EXOGENOUS DNA AND A SELECTABLE MARKER GENE BY ELECTROPORATION

Exponentially growing or early stationary phase fibroblasts are trypsinized and rinsed from the plastic surface with nutrient medium. An aliquot of the cell suspension is removed for counting, and the remaining cells are subjected to centrifugation. The supernatant is aspirated and the pellet is resuspended in 5 ml of electroporation buffer (20 mM HEPES pH 7.3, 137 mM NaCl, 5 mM KCl, 0.7 mM Na₂HPO₄, 6 mM dextrose). The cells are recentrifuged, the supernatant aspirated, and the cells resuspended in electroporation buffer containing 1 mg/ml acetylated bovine serum albumin. The final cell suspension contains approximately 3 x 10⁶ cells/ml. Electroporation should be performed immediately following resuspension.

Supercoiled plasmid DNA is added to a sterile cuvette with a 0.4 cm electrode gap (Bio-Rad.) The final DNA concentration is generally at least 120 µg/ml. 0.5 ml of the cell suspension (containing approximately 1.5 x 10⁶ cells) is then added to the cuvette, and the cell suspension and DNA solutions are gently mixed. Electroporation is performed with a Gene-Pulser apparatus (Bio-Rad). Capacitance and voltage are set at 960 µF and 250-300 V, respectively. As voltage increases, cell survival decreases, but the percentage of surviving cells that stably incorporate the introduced DNA into their genome increases dramatically. Given these parameters, a pulse time of approximately 14-20 mSec should be observed.

Electroporated cells are maintained at room temperature for approximately 5 min, and the contents of the cuvette are then gently removed with a sterile transfer pipette. The cells are added directly to 10 ml of prewarmed nutrient media (as above with 15% calf serum) in a 10 cm dish and incubated as described above. The following day, the media is aspirated and replaced with 10 ml of fresh media and incubated for a further 16-24 hours. Subculture of cells to determine cloning efficiency and to select for G418-resistant colonies is performed the following day. Cells are trypsinized, counted and plated; typically, fibroblasts are plated at 10³ cells/10 cm dish for the determination of cloning efficiency and at 1-2 x 10⁴ cells/10 cm dish for G418 selection.

Human fibroblasts are selected for G418 resistance in medium consisting of 300-400 µg/ml G418 (Geneticin, disulfate salt with a potency of approximately 50%; Gibco) in fibroblasts nutrient media (with 15% calf serum). Cloning efficiency is determined in the absence of G418. The plated cells are incubated for 12-14 days, at which time colonies are fixed with formalin, stained with crystal violet and counted (for cloning efficiency plated) or isolated using cloning cylinders (for G418 plates). Electroporation and selection of rabbit fibroblasts is performed essentially as described for human fibroblasts, with the exception of the selection conditions used. Rabbit fibroblasts are selected for G418 resistance in medium containing 1 gm/ml G418.

Fibroblasts were isolated from freshly excised human foreskins. Cultures were seeded at 50,000 cells/cm in DMEM + 10% calf serum. When cultures became confluent, fibroblasts were harvested by trypsinization and transfected by electroporation. Electroporation conditions were evaluated by transfection with the plasmid pcDNEO. A representative electroporation experiment using near optimal conditions (60 µg of plasmid pcDNEO at an electroporation voltage of 250 volts and a capacitance setting of 960 µFarads) resulted in one G418 colony per 588 treated cells (0.17% of all cells treated), or one G418 colony per 71 clonable cells(1.4%).

When nine separate electroporation experiments at near optimal conditions (60 µg of plasmid pcDNEO at an electroporation voltage of 300 volts and a capacitance setting of 960 µFarads) were performed, an average of one G418 colony per 1,899 treated cells (0.05%) was observed, with a range of 1/882 to 1/7,500 treated cells. This corresponds to an average of one G418 colony per 38 clonable cells (2.6%).

Low passage primary human fibroblasts were converted to hGH expressing cells by co-transfection with plasmids; pcDNEO and pXGH5. Typically, 60 µg of an equimolar mixture of the two plasmids were transfected at near optimal conditions (electroporation voltage of 300 volts and a capacitance setting of 960 µFarads). The results of such an experiment resulted in one G418 colony per 14,705 treated cells.

hGH expression data for these and other cells isolated under identical transfection conditions are summarized below. Ultimately, 98% of all G418^{r} colonies could be expanded to generate mass cultures.

| | |
|---|---|
| Number of G418^{r} Clones | |
| Analyzed | 154 |
| Number of G418^{r}/hGH | |
| Expressing Clones | 65 |
| Average hGH Expression | |
| Level | 2.3 µg hGH/10⁶ Cells/24 hr |
| Maximum hGH Expression | |
| Level | 23.0 µg hGH/10⁶ Cells/24 hr |

Stable transfectants also have been generated by electroporation of primary or secondary human fibroblasts with pXGH301, a DNA construct in which the neo and hGH genes are present on the same plasmid molecule. pXGH301 was constructed by a two-step procedure. The SaII-ClaI fragment from pBR322 (positions 23-651 in pBR322) was isolated and inserted into SaII-ClaI digested pcDNEO, introducing a BamHI site upstream of the SV40 early promoter region of pcDNEO. This plasmid, pBNEO was digested with BamHi and the 2.1 kb fragment containing the neo gene under the control of the SV40 early promoter, was isolated and inserted into BamHI digested pXGH5. A plasmid with a single insertion of the 2.1 kb BamHI fragment was isolated in which neo and hGH are transcribed in the same direction relative to each other. This plasmid was designated pXGH301. For example, 1.5 x 10⁶ cells were electroporated with 60 µg pXGH301 at 300 volts and 960 µFarads. G418 resistant colonies were isolated from transfected secondary fibroblasts at a frequency of 652 G418 resistant colonies per 1.5 x 10 treated cells (1 per 2299 treated cells). Approximately 59% of these colonies express hGH.

### EXAMPLE 2. CONSTRUCTION OF TARGETING PLASMIDS WHICH RESULT IN CHIMERIC TRANSCRIPTION UNITS IN WHICH HUMAN GROWTH HORMONE AND ERYTHROPOIETIN SE-OUENCES ARE FUSED

The following serves to illustrate two further embodiments of the present invention, in which the normal regulatory sequences upstream of the human EPO gene are altered to allow expression of hEPO in primary or secondary fibroblast strains which do not express EPO in detectable quantities in their untransfected state as obtained. In these embodiments, the products of the targeting events are chimeric transcription units in which the first exon of the human growth hormone gene is positioned upstream of EPO exons 2-5. The product of transcription, splicing and translation is a protein in which amino acids 1-4 of the hEPO signal peptide are replaced with amino acid residues 1-3 of hGH. The two embodiments differ with respect to both the relative positions of the foreign regulatory sequences that are inserted and the specific pattern of splicing that needs to occur to produce the final, processed transcript.

Plasmid pXEPO-10 is designed to replace exon 1 of hEPO with exon 1 of hGH by gene targeting to the endogenous hEPO gene on human chromosome 7. Plasmid pXEPO-10 is constructed as follows. First, the intermediate plasmid pT163 is constructed by inserting the 6 kb HindIII-BamHI fragment (see Example 1f) lying upstream of the hEPO coding region into HindIII-BamHI digested pBluescriptII SK+ (Stratagene, LaJolla, CA). The product of this ligation is digested with XhoI and HindIII and ligated to the 1.1 kb HindIII-XhoI fragment from pMClneoPolyA [Thomas, K. R. and Capecchi, M. R. Cell 51: 503-512 (1987) available from Strategene, LaJolla, CA] to create pT163. Oligonucleotides 13.1 - 13.4 are utilized in polymerase chain reactions to generate a fusion fragment in which the mouse metallothionein 1 (mMT-I) promoter - hGH exon 1 sequences are additionally fused to hEPO intron 1 sequences. First, oligonucleotides 13.1 and 13.2 are used to amplify the approximately 0.73 kb mMT-I promoter - hGH exon 1 fragment from pXGH5 (Figure 1). Next, oligonucleotides 13.3 and 13.4 are used to amplify the approximately 0.57 kb fragment comprised predominantly of hEPO intron 1 from human genomic DNA. Finally, the two amplified fragments are mixed and further amplified with oligonucleotides 13.1 and 13.4 to generate the final fusion fragment (fusion fragment 3) flanked by a SalI site at the 5' side of the mMT-I moiety and an XhoI site at the 3' side of the hEPO intron 1 sequence. Fusion fragment 3 is digested with XhoI and SalI and ligated to XhoI digested pT163. The ligation mixture is transformed into E. coli and a clone containing a single insert of fusion fragment 3 in which the XhoI site is regenerated at the 3' side of hEPO intron 1 sequences is identified and designated pXEPO-10. 
13.1 5' AAAAGTCGAC GGTACCTTGG TTTTTAAAAC CAGCCTGGAG
SalI KpnI
(SEQ ID NO 5)
 13.2 5' CC**TAG**CGGC**A AT**GGCTACAG GTGAGTACTC GCGGGCTGGG CG (SEQ ID NO 6)
13.3 5' CGCCCAGCCC GCGAGTACTC ACCTG**TA**GCC **AT**TGCCGC**TA** GG (SEQ ID NO 7)

13.4 5' TTTTCTCGAG CTAGAACAGA TAGCCAGGCT GAGAG
XhoI
(SEQ ID NO 8)

The non-boldface region of oligo 13.1 is identical to the mMT-I promoter, with the natural KpnI site as its 5' boundary. The boldface type denotes a SalI site tail to convert the 5' boundary to a SalI site. The boldface region of oligos 13.2 and 13.3 denote hGH sequences, while the non-boldface regions are intron 1 sequences from the hEPO gene. The non-boldface region of oligo 13.4 is identical to the last 25 bases of hEPO intron 1. The boldface region includes an XhoI site tail to convert the 3' boundary of the amplified fragment to an XhoI site.

Plasmid pXEPO-11 is designed to place, by gene targeting, the mMT-I promoter and exon 1 of hGH upstream of the hEPO structural gene and promoter region at the endogenous hEPO locus on human chromosome 7. Plasmid pXEPO-11 is constructed as follows. Oligonucleotides 13.1 and 13.5 - 13.7 are utilized in polymerase chain reactions to generate a fusion fragment in which the mouse metallothionein I (mMT-I) promoter - hGH exon 1 sequences are additionally fused to hEPO sequences from -1 to -630 relative to the hEPO coding region. First, oligonucleotides 13.1 and 13.5 are used to amplify the approximately 0.73 kb mMT-I promoter - hGH exon 1 fragment from pXGH5 (Figure 1). Next, oligonucleotides 13.6 and 13.7 are used to amplify, from human genomic DNA, the approximately 0.62 kb fragment comprised predominantly of hEPO sequences from -1 to -620 relative to the hEPO coding region. Both oligos 13.5 and 13.6 contain a 10 bp linker sequence located at the hGH intron 1 - hEPO promoter region, which corresponds to the natural hEPO intron 1 splice donor site. Finally, the two amplified fragments are mixed and further amplified with oligonucleotides 13.1 and 13.7 to generate the final fusion fragment (fusion fragment 6) flanked by a SalI site at the 5' side of the mMT-I moiety and an XhoI site at the 3' side of the hEPO promoter region. Fusion fragment 6 is digested with XhoI and SalI and ligated to XhoI digested pT163. The ligation mixture is transformed into E. coli and a clone containing a single insert of fusion fragment 6 in which the XhoI site is regenerated at the 3' side of hEPO promoter sequences is identified and designated pXEPO-11.

13.5    5' CCTAGCGGCA ATGGCTACAG GTGAGTACTC AAGCTTCTGG
                                                    HindIII
        GCTTCCAGAC CCAG  (SEQ ID NO 9)

13.6 5' CTGGGTCTGG AAGCCCAGAA GCTTGAGTAC TCACCTGTAG
HindIII
CCATTGCCGC TAGG (SEQ ID NO 10)

13.7 5' TTTTCTCGAG CTCCGCGCCT GGCCGGGGTC CCTC
XhoI
(SEQ ID NO 11)

The boldface regions of oligos 13.5 and 13.6 denote hGH sequences. The italicized regions correspond to the first 10 base pairs of hEPO intron 1. The remainder of the oligos correspond to hEPO sequences from -620 to -597 relative to the hEPO coding region. The non-boldface region of oligo 13.7 is identical to bases -1 to -24 relative to the hEPO coding region. The boldface region includes an XhoI site tail to convert the 3' boundary of the amplified fragment to an XhoI site.

Plasmid pXEPO-10 can be used for gene targeting by digestion with BamHI and XhoI to release the 7.3 kb fragment containing the mMT-I/hGH fusion flanked on both sides by hEPO sequences. This fragment (targeting fragment 1) contains no hEPO coding sequences, having only sequences lying between -620 and approximately -6620 upstream of the hEPO coding region and hEPO intron 1 sequences to direct targeting to the human EPO locus. Targeting fragment 1 is transfected into primary or secondary human skin fibroblasts using conditions similar to those described in Example 1c. G418-resistant colonies are picked into individual wells of 96-well plates and screened for EPO expression by an ELISA assay (R&D Systems, Minneapolis MN). Cells in which the transfecting DNA integrates randomly into the human genome cannot produce EPO. Cells in which the transfecting DNA has undergone homologous recombination with the endogenous hEPO intron 1 and hEPO upstream sequences contain a chimeric gene in which the mMT-I promoter and non-transcribed sequences and the hGH 5' untranslated sequences and hGH exon 1 replace the normal hEPO promoter and hEPO exon 1 (see Figure 5). Non-hEPO sequences in targeting fragment 1 are joined to hEPO sequences down-stream of hEPO intron 1. The replacement of the normal hEPO regulatory region with the mMT-I promoter will activate the EPO gene in fibroblasts, which do not normally express EPO. The replacement of hEPO exon 1 with hGH exon 1 results in a protein in which the first 4 amino acids of the hEPO signal peptide are replaced with amino acids 1-3 of hGH, creating a functional, chimeric signal peptide which is removed by post-translation processing from the mature protein and is secreted from the expressing cells.

Plasmid pXEPO-11 can be used for gene targeting by digestion with BamHI and XhoI to release the 7.4 kb fragment containing the mMT-I/hGH fusion flanked on both sides by hEPO sequences. This fragment (targeting fragment 2) contains no hEPO coding sequences, having only sequences lying between -1 and approximately -6620 upstream of the hEPO coding region to direct targeting to the human EPO locus. Targeting fragment 2 is transfected into primary or secondary human skin fibroblasts using conditions similar to those described in Example 1g. G418-resistant colonies are picked into individual wells of 96-well plates and screened for EPO expression by an ELISA assay (R&D Systems, Minneapolis, MN). Cells in which the transfecting DNA integrates randomly into the human genome cannot produce EPO. Cells in which the transfecting DNA has undergone homologous recombination with the endogenous hEPO promoter and upstream sequences contain a chimeric gene in which the mMT-I promoter and non-transcribed sequences, hGH 5' untranslated sequences and hGh exon 1, and a 10 base pair linker comprised of the first 10 bases of hEPO intron 1 are inserted at the HindIII site lying at position -620 relative to the hEPO coding region (see Figure 6). The localization of the mMT-I promoter upstream of the normally silent hEPO promoter will direct the synthesis, in primary or secondary skin fibroblasts, of a message reading (5' to 3') non-translated metallothionein and hGH sequences, hGH exon 1, 10 bases of DNA identical to the first 10 base pairs of hEPO intron 1, and the normal hEPO promoter and hEPO exon 1 (-620 to +13 relative to the EPO coding sequence). The 10 base pair linker sequence from hEPO intron 1 acts as a splice donor site to fuse hGH exon 1 to the next downstream splice acceptor site, that lying immediately upstream of hEPO exon 2. Processing of the resulting transcript will therefore splice out the hEPO promoter, exon 1, and intron 1 sequences. The replacement of hEPO exon 1 with hGH exon 1 results in a protein in which the first 4 amino acids of the hEPO signal peptide are replaced with amino acids 1-3 of hGH, creating a functional, chimeric signal peptide which is removed by post-translation processing from the mature protein and is secreted from the expressing cells.

A series of constructs related to pXEPO-10 and pXEPO-11 can be constructed, using known methods. In these constructs, the relative positions of the mMT-I promoter and hGH sequences, as well as the position at which the mMT-I/hGH sequences are inserted into hEPO upstream sequences, are varied to create alternative chimeric transcription units that facilitate gene targeting, result in more efficient expression of the fusion transcripts, or have other desirable properties. Such constructs will give similar results, such that an hGH-hEPO fusion gene is placed under the control of an exogenous promoter by gene targeting to the normal hEPO locus. For example, the 6 kb HindIII-BamHI fragment upstream of the hEPO gene (See Example 1f) has numerous restriction enzyme recognition sequences that can be utilized as sites for insertion of the neo gene and the mMT-I promoter/hGH fusion fragment. One such site, a BglII site lying approximately 1.3 kb upstream of the HindIII site, is unique in this region and can be used for insertion of one or more selectable markers and a regulatory region derived from another gene that will serve to activate EPO expression in primary, secondary, or immortalized human cells.

First, the intermediate plasmid pT164 is constructed by inserting the 6 kb HindIII-BamHI fragment (Example 1f) lying upstream of the hEPO coding region into HindIII-BamHI digested pBluescriptII SK+ (Stratagene, LaJolla, CA). Plasmid pMClneoPolyA [Thomas, K.R. and Capecchi, M.R. Cell 51:503-512 (1987); available from Stratagene, LaJolla, CA] is digested with BamHI and XhoI, made blunt-ended by treatment with the Klenow fragment of E. coli DNA polymerase, and the resulting 1.1 kb fragment is purified. pT164 is digested with BglII and made blunt-ended by treatment with the Klenow fragment of E. coli DNA polymerase. The two preceding blunt-ended fragments are ligated together and transformed into competent E. coli. Clones with a single insert of the 1.1 kb neo fragment are isolated and analyzed by restriction enzyme analysis to identify those in which the BglII site recreated by the fusion of the blunt XhoI and BglII sites is localized 1.3 kb away from the unique HindIII site present in plasmid pT164. The resulting plasmid, pT165, can now be cleaved at the unique BglII site flanking the 5' side of the neo transcription unit.

Oligonucleotides 13.8 and 13.9 are utilized in polymerase chain reactions to generate a fragment in which the mouse metallothionein I (mMT-I) promoter - hGH exon 1 sequences are additionally fused to a 10 base pair fragment comprising a splice donor site. The splice donor site chosen corresponds to the natural hEPO intron 1 splice donor site, although a larger number of splice donor sites or consensus splice donor sites can be used. The oligonucleotides (13.8 and 13.9) are used to amplify the approximately 0.73 kb mMT-1 promoter - hGH exon 1 fragment from pXGH5 (Figure 1). The amplified fragment (fragment 7) is digested with BglII and ligated to BglII digested pT165. The ligation mixture is transformed into E. coli and a clone, containing a single insert of fragment 7 in which the KpnI site in the mMT-I promoter is adjacent to the 5' end of the neo gene and the mMT-I promoter is oriented such that transcription is directed towards the unique HindIII site, is identified and designated pXEPO-12.

13.8 5' AAAAAGATCT GGTACCTTGG TTTTTAAAAC CAGCCTGGAG
BglII KpnI
(SEQ ID NO 12)

The non-boldface region of oligo 13.8 is identical to the mMT-I promoter, with the natural KpnI site as its 5' boundary. The boldface type denotes a BglII site tail to convert the 5' boundary to a BglII site.

13.9 5' TTTTAGATCT GAGTACTCAC CTGTAGCCAT TGCCGCTAGG
BglII
(SEQ ID NO 13)

The boldface region of oligos 13.9 denote hGH sequences. The italicized region corresponds to the first 10 base pairs of hEPO intron 1. The underlined BglII site is added for plasmid construction purposes.

Plasmid pXEPO-12 can be used for gene targeting by digestion with BamHI and HindIII to release the 7.9 kb fragment containing the neo gene and the mMT-I/hGH fusion flanked on both sided by hEPO sequences. This fragment (targeting fragment 3) contains no hEPO coding sequences, having only sequences lying between approximately -620 and approximately -6620 upstream of the hEPO coding region to direct targeting upstream of the human EPO locus. Targeting fragment 3 is transfected into primary, secondary, or immortalized human skin fibroblasts using conditions similar to those described in Examples 1b and 1c. G418-resistant colonies are picked into individual wells of 96-well plates and screened for EPO expression by an ELISA assay (R&D Systems, Minneapolis MN). Cells in which .the transfecting DNA integrates randomly into the human genome cannot produce EPO. Cells in which the transfecting DNA has undergone homologous recombination with the endogenous hEPO promoter and upstream sequences contain a chimeric gene in which the mMT-I promoter and non-transcribed sequences, hGH 5' untranslated sequences, and hGH exon 1, and a 10 base pair linker comprised of the first 10 bases of hEPO intron 1 are inserted at the BglII site lying at position approximately -1920 relative to the hEPO coding region. The localization of the mMT-I promoter upstream of the normally silent hEPO promoter will direct the synthesis, in primary, secondary, or immortalized human fibroblasts (or other human cells), of a message reading: (5' to 3') nontranslated metallothionein and hGH sequences, hGH exon 1, 10 bases of DNA identical to the first 10 base pairs of hEPO intron 1, and hEPO upstream region and hEPO exon 1 (from approximately -1920 to +13 relative to the EPO coding sequence). The 10 base pair linker sequence from hEPO intron 1 acts as a splice donor site to fuse hGH exon 1 to a downstream splice acceptor site, that lying immediately upstream of hEPO exon 2. Processing of the resulting transcript will therefore splice out the hEPO upstream sequences, promoter region, exon 1, and intron 1 sequences. When using pXEPO-10, -11 and -12, post-transcriptional processing of the message can be improved by using in vitro mutagenesis to eliminate splice acceptor sites lying in hEPO upstream sequences between the mMT-I promoter and hEPO exon 1, which reduce level of productive splicing events needed create the desired message. The replacement of hEPO exon 1 with hGH exon 1 results in a protein in which the first 4 amino acids of the hEPO signal peptide are replaced with amino acids 1-3 of hGH, creating a functional, chimeric signal peptide which is removed by post-translation processing from the mature protein and is secreted from the expressing cells.

### EXAMPLE 3. TARGETED MODIFICATION OF SEOUENCES_UPSTREAM AND AMPLIFICATION OF THE TARGETED GENE

Human cells in which the EPO gene has been activated by the methods previously described can be induced to amplify the neo/mMT-1/EPO transcription unit if the targeting plasmid contains a marker gene that can confer resistance to a high level of a cytotoxic agent by the phenomenon of gene amplification. Selectable marker genes such as dihydrofolate reductase (dhfr, selective agent is methotrexate), the multifunctional CAD gene [encoding carbamyl phosphate synthase, aspartate transcarbamylase, and dihydro-orotase; selective agent is N-(phosphonoacetyl)-L-aspartate (PALA)], and adenosine deaminase (ada; selective agent is an adenine nucleoside), have been documented, among other genes, to be amplifiable in immortalized human cell lines (Wright, J.A. et al. Proc. Natl. Acad. Sci. USA 87:1791-1795 (1990)). In these studies, gene amplification has been documented to occur in a number of immortalized human cell lines. HT1080, HeLa, MCF-7 breast cancer cells, K-562 leukemia cells, KB carcinoma cells, or 2780AD ovarian carcinoma cells all display amplification under appropriate selection conditions.

Plasmids pXEPO-10 and pXEPO-11 can be modified by the insertion of a normal or mutant dhfr gene into the unique HindIII sites of these plasmids. After transfection of HT1080 cells with the appropriate DNA, selection for G418-resistance (conferred by the neo gene), and identification of cells in which the hEPO gene has been activated by gene targeting of the neo, dhfr, and mMT-1 sequences to the correct position upstream of the hEPO gene, these cells can be exposed to stepwise selection in methotrexate (MTX) in order to select for amplification of dhfr and co-amplification of the linked neo, mMT-1, and hEPO sequences (Kaufman, R.J. Technique 2:221-236 (1990)). A stepwise selection scheme in which cells are first exposed to low levels of MTX (0.01 to 0.08 µM), followed by successive exposure to incremental increases in MTX concentrations up to 250 µM MTX or higher is employed. Linear incremental steps of 0.04 to 0.08 µM MTX and successive 2-fold increases in MTX concentration will be effective in selecting for amplified transfected cell lines, although a variety of relatively shallow increments will also be effective. Amplification is monitored by increases in dhfr gene copy number and confirmed by measuring in vitro hEPO expression. By this strategy, substantial over-expression of hEPO can be attained by targeted modification of sequences lying completely outside of the hEPO coding region.

Constructs similar to those described (Examples 1i and lk) to activate hGH expression in human cells can also be further modified to include the dhfr gene for the purpose of obtaining cells that overexpress the hGH gene by gene targeting to non-coding sequences and subsequent amplification.

### EXAMPLE 4. TARGETING AND ACTIVATION OF THE HUMAN EPO LOCUS IN AN IMMORTALIZED HUMAN FIBROBLAST LINE

The targeting construct pXEPO-13 was made to test the hypothesis that the endogenous hEPO gene could be activated in a human fibroblast cell. First, plasmid pT22.1 was constructed, containing 63 bp of genomic hEPO sequence upstream of the first codon of the hEPO gene fused to the mouse metallothionein-1 promoter (mMT-I). Oligonucleotides 22.1 to 22.4 were used in PCR to fuse mMT-I and hEPO sequences. The properties of these primers are as follows: 22.1 is a 21 base oligonucleotide homologous to a segment of the mMT-I promoter beginning 28 bp upstream of the mMT-I KpnI site; 22.2 and 22.3 are 58 nucleotide complementary primers which define the fusion of hEPO and mMT-I sequences such that the fusion contains 28 bp of hEPO sequence beginning 35 bases upstream of the first codon of the hEPO gene, and mMT-I sequences beginning at base 29 of oligonucleotide 22.2, comprising the natural BglII site of mMT-I and extending 30 bases into mMT-I sequence; 22.4 is 21 nucleotides in length and is homologous to hEPO sequences beginning 725 bp downstream of the first codon of the hEPO gene. These primers were used to amplify a 1.4 kb DNA fragment comprising a fusion of mMT-I and hEPO sequences as described above. The resulting fragment was digested with KpnI (the PCR fragment contained two KpnI sites: a single natural KpnI site in the mMT-I promoter region and a single natural KpnI site in the hEPO sequence), and purified. The plasmid pXEPO1 (Figure 3) was also digested with KpnI, releasing a 1.4 kb fragment and a 6.4 kb fragment. The 6.4 kb fragment was purified and ligated to the 1.4 kb KpnI PCR fusion fragment. The resulting construct was called pT22.1. A second intermediate, pT22.2, was constructed by ligating the approximately 6 kb HindIII-BamHI fragment lying upstream of the hEPO structural gene (see Example 1f) to BamHI and HindIII digested pBSIISK+ (Stratagene, LaJolla, CA). A third intermediate, pT22.3, was constructed by first excising a 1.1 kb XhoI/BamHI fragment from pMCINEOpolyA (Stratagene,, LaJolla, CA) containing the neomycin phosphotransferase gene. The fragment was then made blunt-ended with the Klenow fragment of DNA polymerase I (New England Biolabs). This fragment was then ligated to the HincII site of pBSIISK+ (similarly made blunt with DNA polymerase I) to produce pT22.3. A fourth intermediate, pT22.4, was made by purifying a 1.1 kb XhoI/HindIII fragment comprising the neo gene from pT22.3 and ligating this fragment to XhoI and HindIII digested pT22.2. pT22.4 thus contains the neo gene adjacent to the HindIII side of the BamHI-HindIII upstream hEPO fragment. Finally, pXEPO-13 was generated by first excising a 2.0 kb EcoRI/AccI fragment from pT22.1. The EcoRI site of this fragment defines the 5' boundary of the mMT-I promoter, while the AccI site of this fragment lies within hEPO exon 5. Thus, the AccI/EcoRI fragment contains a nearly complete hEPO expression unit, missing only a part of exon 5 and the natural polyadenylation site. This 2.0 kb EcoRI/AccI fragment was purified, made blunt-ended by treatment with the Klenow fragment of DNA polymerase I, and ligated to XhoI digested, blunt-ended, pT22.4.

HT1080 cells were transfected with PvuI-BamHI digested pXEPO-13. pXEPO-13 digested in this way generates three fragments; a 1 kb vector fragment including a portion of the amp gene, a 1.7 kb fragment of remaining vector sequences and an approximately 10 kb fragment containing hEPO, neo and mMT-I sequences. This approximately 10 kb BamHI/PvuI fragment contained the following sequences in order from the BamHI site: an approximately 6.0 kb of upstream hEPO genomic sequence, the 1.1 kb neo transcription unit, the 0.7 kb mMT-1 promoter and the 2.0 kb fragment containing hEPO coding sequence truncated within exon 5. 45µg of pEXPO-13 digested in this way was used in an electroporation of 12 million cells (electroporation conditions were described in Example 1b). This electroporation was repeated a total of eight times, resulting in electroporation of a total of 96 million cells. Cells were mixed with media to provide a cell density of 1 million cells per ml and 1 ml aliquots were dispensed into a total of 96, 150mm tissue culture plates (Falcon) each containing a minimum of 35 ml of DMEM/15% calf serum. The following day, the media was aspirated and replaced with fresh medium containing 0.8 mg/ml G418 (Gibco). After 10 days of incubation, the media of each plate was sampled for hEPO by ELISA analysis (R & D Systems). Six of the 96 plates contained at least 10 mU/ml hEPO. One of these plates, number 18, was selected for purification of hEPO expressing colonies. each of the 96, 150 mm plates contained approximately 600 G418 resistant colonies (an estimated total of 57,600 G418 resistant colonies on all 96 plates). The approximately 600 colonies on plate number 18 were trypsinized and replated at 50 cells/ml into 364 well plates (Sterilin). After one week of incubation, single colonies were visible at approximately 10 colonies per large well of the 364 well plates (these plates are comprised of 16 small wells within each of the 24 large wells). Each well was screened for hEPO expression at this time. Two of the large wells contained media with at least 20 mU/ml hEPO. Well number A2 was found to contain 15 colonies distributed among the 16 small wells. The contents of each of these small wells were trypsinized and transferred to 16 individual wells of a 96 well plate. following 7 days of incubation the media from each of these wells was sampled for hEPO ELISA analysis. Only a single well, well number 10, contained hEPO. This cell strain was designated HT165-18A2-10 and was expanded in culture for quantitative hEPO analysis, RNA isolation and DNA isolation. Quantitative measurement of hEPO production resulted in a value of 2,500 milliunits/million cells/24 hours.

A 0.2 kb DNA probe extending from the AccI site in hEPO exon 5 to the BglII site in the 3' untranslated region was used to probe RNA isolated from HT165-18A2-10 cells. The targeting construct, pXEPO-13, truncated at the AccI site in exon 5 does not contain these AccI/BglII sequences and, therefore, is diagnostic for targeting at the hEPO locus. Only cell strains that have recombined in a homologous manner with natural hEPO sequences would produce an hEPO mRNA containing sequence homologous to the AccI/BglII sequences. HT165-18A2-10 was found to express an mRNA of the predicted size hybridizing with the 32-P labeled AccI/BglII hEPO probe on Northern blots. Restriction enzyme and Southern blot analysis confirmed that the neo gene and mMT-I promoter were targeted to one of the two hEPO alleles in HT165-18A2-10 cells.

These results demonstrate that homologous recombination can be used to target a regulatory region to a gene that is normally silent in human fibroblasts, resulting in the functional activation of that gene.
22.1 5' CACCTAAAAT GATCTCTCTG G (SEQ ID NO 14) 
22.2 5' CGCGCCGGGT GACCACACCG GGGGCCCTAG ATCTGGTGAA
GCTGGAGCTA CGGAGTAA (SEQ ID NO 15)
 
22.3 5' TTACTCCGTA GCTCCAGCTT CACCAGATCT AGGGCCCCCG
GTGTGGTCAC CCGGCGCG (SEQ ID NO 16)
 22.4 5' GTCTCACCGT GATATTCTCG G (SEQ ID NO 17)

### EXAMPLE 5. ACTIVATION AND AMPLIFICATION OF THE EPO GENE IN AN IMMORTALIZE HUMAN CELL LINE

Incorporation of a dhfr expression unit into the unique HindIII site of pXEPO-13 (see Example 4) results in a new targeting vector capable of dual selection and selection of cells in which the dhfr gene is amplified. The single HindIII site in pXEPO-13 defines the junction of the neo gene and genomic sequence naturally residing upstream of the human EPO gene. Placement of a dhfr gene at this site provides a construct with the neo and dhfr genes surrounded by DNA sequence derived from the natural EPO locus. Like pXEPO-13, is useful to target to the EPO locus by homologous recombination. Such a construct designated pREPO4, is represented in Figure 8. The plasmid includes exons 1-4 and part of exon 5 of the human EPO gene, as well as the HindIII-BamHI fragment lying upstream of the hEPO coding region. pSVe, pTK and pmMT-I correspond to the promoters from the SV40 early region, the Herpes Simplex Virus (HSV) thymidine kinase (TK) gene and the mouse metallothionein-I gene. It was produced as follows: HindIII-digested pXEPO-13 was purified and made blunt with the Klenow fragment of DNA polymerase I. To obtain a dhfr expression unit, the plasmid construct pF8CIS9080 (Eaton et al., Biochemistry 25:8343-8347 (1986)) was digested with EcoRI and SalI. A 2 Kb fragment containing the dhfr expression unit was purified from this digest and made blunt with Klenow fragment of DNA polymerase I. This dhfr-containing fragment was then ligated to the blunted HindIII site of pXEPO-13. An aliquot of this ligation was transformed into E. coli and plated on ampicillin selection plates. Following an overnight incubation at 37°C, individual bacterial colonies were observed, picked and grown. Miniplasmid preparations were made from these cultures and the resulting DNA was then subjected to restriction enzyme digestion with the enzymes BglI+HindIII, and SfiI in order to determine the orientation of the inserted dhfr fragments. Plasmid DNA from one of these preparations was found to contain such a 2 Kb insertion of the dhfr fragment. The transcription orientation of the dhfr expression unit in this plasmid was found to be opposite that of the adjacent neo gene. This is the construct designated pREPO4.

Plasmid pREPO4 was used to amplify the hEPO locus in cells subsequent to activation of the endogenous hEPO gene by homologous recombination. Gene activation with this construct allows selection for increased DHFR expression by the use of the drug methotrexate (MTX). Typically, increased DHFR expression would occur by an increase in copy number through DNA amplification. The net result would be co-amplification of the activated EPO gene along with dhfr sequences. Co-amplification of the activated EPO locus should result in increased EPO expression.

Targeting experiments were performed in HT1080 cells with pREPO4. hEPO expressing line HTREPO-52 was isolated. This line was analyzed quantitatively for EPO production and by Southern and Northern blot. Both strains were found to be targeted with a single copy of dhfr/neo/mMT-1 sequences. Expression levels obtained under 0.8 mg/ml G418 selection were approximately 1300 mU/million cells/day. Because the targeted EPO locus contained a dhfr expression unit, it was possible to select for increased expression of DHFR with the antifolate drug, MTX. These strains were therefore subjected to stepwise selection in 0.02, 0.05, 0.1, 0.2 and 0.4 µM MTX. Results of initial selection of mass cultures of these strains are shown in Table 4 and Figure 7.

**TABLE 4**

| Cell Line | MTX(µM) | mU/ Million Cells/ 24h |
|---|---|---|
| 52C20-5-0 | 0 | 1368 |
| 52C20-5-.01 | 0.01 | 1744 |
| 52C20-5-.02 | 0.02 | 11643 |
| 52C20-5-0.05 | 0.05 | 24449 |
| 52-3-5-0.10 | 0.1 | 37019 |
| 52-3-2-0.20 | 0.2 | 67867 |
| 52-3-2-0.4B | 0.4 | 99919 |

Selection with elevated levels of MTX was successful in increasing EPO expression in line HTREPO-52, with a 70-fold increase in EPO production seen in the cell line resistant to 0.4 µM MTX. Confirmation of amplification of the EPO locus was accomplished by Southern blot analysis in MTX-resistant cell lines, which revealed an approximately 10-fold increase in the copy number of the activated hEPO locus relative to the parental (untargeted) hEPO allele.

## Claims

1. An *in vitro* gene amplification method of altering the expression of an endogenous gene of a vertebrate cell comprising the steps of:
a) transfecting the cell with a DNA construct comprising:
1) an exogenous DNA regulatory sequence that is not normally functionally linked to the endogenous gene in the cell as obtained;
2) one or more targeting sequences that are homologous with one or more genomic DNA sequences at a preselected site in the cell;
3) an amplifiable DNA sequence encoding a selectable marker; and
4) a sequence encoding an unpaired splice donor site;
b) maintaining the cell produced in a) under conditions appropriate for homologous recombination to occur between the one or more DNA sequences specified in a) 2) above and the one or more genomic sequences at the preselected site, thereby producing a homologously recombinant cell of vertebrate origin having the DNA sequences of a) 1), a) 3) and a) 4) integrated into genomic DNA such that transcription can occur under the control of the regulatory sequence in a manner so that the first exon of the endogenous gene is spliced out;
and
c) culturing the homologously recombinant cell produced in b) under conditions that select for amplification of the amplifiable DNA encoding a selectable marker and that allow the altered expression of the endogenous gene, whereby the amplifiable DNA encoding a selectable marker and the exogenous DNA regulatory sequence functionally linked to DNA of the endogenous gene are coamplified and said altered expression is obtained.

2. The method of claim 1, wherein the vertebrate cell is a primary or secondary cell.

3. The method of claim 2, wherein the primary or secondary cell is a mammalian cell.

4. The method of claim 2 or 3, wherein the primary or secondary cell is a human cell.

5. The method of any preceding claim, wherein the vertebrate cell is an immortalised cell.

6. The method according to any of claims 1 to 5, wherein the cell is selected from the group consisting of: HT1080 cells, HeLa cells, MCF-7 breast cancer cells, K-562 leukemia cells, KB carcinoma cells, 2780AD ovarian carcinoma cells, Raji cells, Jurkat cells, Namalwa cells, HL-60 cells, Daudi cells, RPMI 8226 cells, MOLT-4 cells, CHO cells and mouse L cells.

7. The method of any one of claims 1 to 5, wherein the amplifiable DNA encoding a selectable marker encodes a selectable marker selected from the group consisting of: dihydrofolate reductase, adenosine deaminase, and CAD.

8. The method according to any of claims 1 to 7, wherein the DNA construct further comprises (a) an additional positive selection marker, (b) a negative selection marker, or (c) an additional positive selection marker and a negative selection marker.

9. The method of claim 8 in which the additional positive selection marker is neo.

10. The method of claim 9 in which the positive selection marker is neo and the negative selection marker is gpt or the HSV-TK gene.

11. The method of any one of claims 1 to 10, wherein the endogenous gene encodes a therapeutic protein.

12. The method of any one of claims 1 to 11, wherein the endogenous gene encodes a hormone, a cytokine, an antigen, an antibody, an enzyme, a clotting factor, a transport protein, a receptor, a regulatory protein, a structural protein or a transcription factor.

13. The method of any of claims 1 to 11, wherein the endogenous gene encodes a therapeutic protein selected from the group consisting of Factor VIII, Factor IX, erythropoietin, alpha-1 antitrypsin, calcitonin, glucocerebrosidase, growth hormone, low density lipoprotein (LDL) receptor, apolipoproteins, IL-2 receptor, IL-2 receptor antagonists, insulin, globins, immunoglobulins, catalytic antibodies, interleukins, insulin-like growth factors, superoxide dismutase, immune response modifiers, parathyroid hormone, interferons, nerve growth factors, tissue plaminogen activators and colony stimulating factors.

14. The method of any one of claims 1 to 11, wherein the endogenous gene encodes a therapeutic protein selected from the group consisting of human growth hormone, human insulin, human insulinotropin, and human erythropoietin.

15. The method according to any preceding claim, wherein said altered expression provides a protein that is not naturally expressed in the vertebrate cell by the endogenous gene.

16. The method according to any preceding claim, wherein said altered expression provides the natural protein product of the endogenous gene following any post-translation processing that occurs in the cell.

17. The method according to any preceding claim, wherein the regulatory sequence is selected from the group consisting of: promoters, enhancers, scaffold-attachment regions, negative-regulatory elements, transcriptional initiation sites and regulatory protein binding sites.

18. The method according to any preceding claim, wherein the construct comprises a combination of regulatory sequences.

19. The method according to any preceding claim, wherein the construct comprises a polyadenylation signal.

20. A homologously recombinant cell produced by a method according to any of claims 1 to 19; wherein said cell comprises a selectable marker as described in claim 7.

21. A device containing a cell according to claim 20 that expresses a therapeutic product, wherein the therapeutic product, but not the cell, is freely permeable through the device.

22. A cell according to claim 20 or a device according to claim 21, for use in medicine.

23. A cell according to claim 20, for use in gene therapy.

24. The use of a cell according to claim 20 in the preparation of a device according to claim 21.

25. A construct that is capable of altering the expression of an endogenous gene of a vertebrate cell and is suitable for use in a method according to any of claims 1 to 19; wherein the construct comprises:
1) an exogenous DNA regulatory sequence that is not normally functionally linked to the endogenous gene;
2) one or more targeting sequences that are homologous with one or more genomic DNA sequences at a preselected site in the cell;
3) an amplifiable DNA sequence encoding a selectable marker; and
4) a sequence encoding an unpaired splice donor site;
wherein the construct is such so that, following homologous recombination between said one or more targeting sequences and said one or more genomic sequences at the preselected site, transcription can occur under the control of the regulatory sequence in a manner so that the first exon of the endogenous gene is spliced out, and wherein the amplifiable DNA encoding the selectable marker and the exogenous DNA regulatory sequence functionally linked to DNA of the endogenous gene can be coamplified;
and wherein said selectable marker is a marker as described in claim 7.

26. A construct according to claim 25, for use in medicine.

27. A construct according to claim 25, for use in gene therapy.

28. The use of a construct according to claim 25 in the preparation of a device according to claim 21.

## Patentansprüche

1. *In*-*vitro*-Genamplifikationsverfahren zur Veränderung der Expression eines endogenen Gens einer Vertebratenzelle, das die folgenden Schritte umfasst:
a) Transfizieren der Zelle mit einem DNA-Konstrukt, umfassend:
1) eine exogene DNA-Regulationssequenz, die normalerweise nicht funktionell mit dem endogenen Gen in der Zelle, wie erhalten, verknüpft ist;
2) eine oder mehrere Zielsteuerungssequenzen, die mit einer oder mehreren genomischen DNA-Sequenzen an einer zuvor ausgewählten Stelle in der Zelle homolog sind;
3) eine amplifizierbare DNA-Sequenz, die einen Selektionsmarker kodiert, und
4) eine Sequenz, die eine ungepaarte Spleiß-Donorsequenz kodiert,
b) Halten der unter a) hergestellten Zelle unter geeigneten Bedingungen, dass eine homologe Rekombination zwischen der (den) vorstehend unter a) 2) angegebenen einen oder mehreren DNA-Sequenzen und der (den) einen oder mehreren genomischen Sequenzen an der zuvor ausgewählten Stelle erfolgt, wodurch eine homolog rekombinierte, aus Vertebraten stammende Zelle hergestellt wird, in der die DNA-Sequenzen aus a) 1), a) 3) und a) 4) in die genomische DNA derart integriert worden sind, dass eine Transkription unter der Kontrolle der Regulationssequenz so erfolgen kann, dass das erste Exon des endogenen Gens herausgespleißt wird,
und
c) Züchten der unter b) hergestellten, homolog rekombinierten Zelle unter Bedingungen, die auf eine Amplifikation der amplifizierbaren DNA selektieren, die einen Selektionsmarker kodiert, und die die veränderte Expression des endogenen Gens gestatten, wodurch die amplifizierbare DNA, die einen Selektionsmarker kodiert, und die exogene DNA-Regulationssequenz, die funktionell mit der DNA des endogenen Gens verknüpft ist, zusammen amplifiziert werden und die veränderte Expression erhalten wird.

2. Verfahren nach Anspruch 1, wobei die Vertebratenzelle eine primäre oder sekundäre Zelle ist.

3. Verfahren nach Anspruch 2, wobei die primäre oder sekundäre Zelle eine Säugerzelle ist.

4. Verfahren nach Anspruch 2 oder 3, wobei die primäre oder sekundäre Zelle eine menschliche Zelle ist.

5. Verfahren nach einem vorhergehenden Anspruch , wobei die Vertebratenzelle eine immortalisierte Zelle ist.

6. Verfahren nach einem der Ansprüche 1 bis 5, wobei die Zelle aus der Gruppe ausgewählt wird, bestehend aus: HT1080-Zellen, HeLa-Zellen, MCF-7-Brustkrebszellen, K-562-Leukämiezellen, KB-Karzinomzellen, 2780AD-Ovarkarzinomzellen, Raji-Zellen, Jurkat-Zellen, Namalwa-Zellen, HL-60-Zellen, Daudi-Zellen, RPMI 8226-Zellen, MOLT-4-Zellen, CHO-Zellen und Maus-L-Zellen.

7. Verfahren nach einem der Ansprüche 1 bis 5, wobei die amplifizierbare DNA, die einen Selektionsmarker kodiert, einen Selektionsmarker kodiert, der aus der Gruppe ausgewählt ist, bestehend aus: Dihydrofolatreduktase, Adenosindesaminase und CAD.

8. Verfahren nach einem der Ansprüche 1 bis 7, wobei das DNA-Konstrukt außerdem (a) einen zusätzlichen positiven Selektionsmarker, (b) einen negativen Selektionsmarker oder (c) einen zusätzlichen positiven Selektionsmarker und einen negativen Selektionsmarker umfasst.

9. Verfahren nach Anspruch 8, wobei es sich bei dem zusätzlichen positiven Selektionsmarker um neo handelt.

10. Verfahren nach Anspruch 9, wobei der positive Selektionsmarker neo ist und der negative Selektionsmarker gpt oder das HSV-TK-Gen ist.

11. Verfahren nach einem der Ansprüche 1 bis 10, wobei das endogene Gen ein therapeutisches Protein kodiert.

12. Verfahren nach einem der Ansprüche 1 bis 11, wobei das endogene Gen ein Hormon, ein Cytokin, ein Antigen, einen Antikörper, ein Enzym, einen Gerinnungsfaktor, ein Transportprotein, einen Rezeptor, ein Regulationsprotein, ein Strukturprotein oder einen Transkriptionsfaktor kodiert.

13. Verfahren nach einem der Ansprüche 1 bis 11, wobei das endogene Gen ein therapeutisches Protein kodiert, das aus der Gruppe ausgewählt ist, bestehend aus: Faktor VIII, Faktor IX, Erythropoetin, alpha-1-Antitrypsin, Calcitonin, Glucocerebrosidase, Wachstumshormon, Low Density Lipoprotein- (LDL-) Rezeptor, Apolipoproteinen, IL-2-Rezeptor, IL-2-Rezeptor-Antagonisten, Insulin, Globinen, Immunglobulinen, katalytischen Antikörpern, Interleukinen, insulinähnlichen Wachstumsfaktoren, Superoxiddismutase, Modifikatoren der Immunantwort, Parathyreoidhormon, Interferonen, Nervenwachstumsfaktoren, Gewebeplasminogenaktivatoren und koloniestimulierenden Faktoren.

14. Verfahren nach einem der Ansprüche 1 bis 11, wobei das endogene Gen ein therapeutisches Protein kodiert, das aus der Gruppe ausgewählt ist, bestehend aus: menschlichem Wachstumshormon, menschlichem Insulin, menschlichem Insulinotropin und menschlichem Erythropoetin.

15. Verfahren nach einem vorhergehenden Anspruch, wobei die veränderte Expression ein Protein liefert, das in der Vertebratenzelle nicht natürlicherweise durch das endogene Gen exprimiert wird.

16. Verfahren nach einem vorhergehenden Anspruch, wobei die veränderte Expression das natürliche Protein des endogenen Gens nach einer beliebigen posttranslationalen Modifikation liefert, die in der Zelle erfolgt.

17. Verfahren nach einem vorhergehenden Anspruch, wobei die Regulationssequenz aus der Gruppe ausgewählt ist, bestehend aus: Promotoren, Enhancern, Gerüstanheftungsregionen, negativen Regulationselementen, Trans-kriptionsinitiationsstellen und Bindungsstellen für Regulationsproteine.

18. Verfahren nach einem vorhergehenden Anspruch, wobei das Konstrukt eine Kombination von Regulationssequenzen umfasst.

19. Verfahren nach einem vorhergehenden Anspruch, wobei das Konstrukt ein Polyadenylierungssignal umfasst.

20. Homolog rekombinierte Zelle, die durch ein Verfahren nach einem der Ansprüche 1 bis 19 hergestellt wurde, wobei die Zelle einen Selektionsmarker nach Anspruch 7 umfasst.

21. Vorrichtung, die eine Zelle nach Anspruch 20 enthält, die ein therapeutisches Produkt exprimiert, wobei das therapeutische Produkt, aber nicht die Zelle, frei durch die Vorrichtung permeieren kann.

22. Zelle nach Anspruch 20 oder Vorrichtung nach Anspruch 21 für die Verwendung in der Medizin.

23. Zelle nach Anspruch 20 für die Verwendung in der Gentherapie.

24. Verwendung einer Zelle nach Anspruch 20 zur Herstellung einer Vorrichtung nach Anspruch 21.

25. Konstrukt, das die Expression eines endogenen Gens einer Vertebratenzelle verändern kann und für die Verwendung bei einem Verfahren nach einem der Ansprüche 1 bis 19 geeignet ist, wobei das Konstrukt Folgendes umfasst:
1) eine exogene DNA-Regulationssequenz, die normalerweise nicht funktionell mit dem endogenen Gen verknüpft ist;
2) eine oder mehrere Zielsteuerungssequenzen, die mit einer oder mehreren genomischen DNA-Sequenzen an einer zuvor ausgewählten Stelle in der Zelle homolog sind;
3) eine amplifizierbare DNA-Sequenz, die einen Selektionsmarker kodiert, und
4) eine Sequenz, die eine ungepaarte Spleiß-Donorsequenz kodiert,
wobei das Konstrukt derart beschaffen ist, dass nach homologer Rekombination zwischen der (den) einen oder mehreren Zielsteuerungssequenzen und der (den) einen oder mehreren genomischen Sequenzen an der zuvor ausgewählten Stelle eine Transkription unter der Kontrolle der Regulationssequenz so erfolgen kann, dass das erste Exon des endogenen Gens herausgespleißt wird, und wobei die amplifizierbare DNA, die den Selektionsmarker kodiert, und die exogene DNA-Regulationssequenz, die funktionell mit der DNA des endogenen Gens verknüpft ist, zusammen amplifiziert werden können,
und wobei der Selektionsmarker ein Marker nach Anspruch 7 ist.

26. Konstrukt nach Anspruch 25 für die Verwendung in der Medizin.

27. Konstrukt nach Anspruch 25 für die Verwendung in der Gentherapie.

28. Verwendung eines Konstrukts nach Anspruch 25 zur Herstellung einer Vorrichtung nach Anspruch 21.

## Revendications

1. Procédé d'amplification génique *in vitro* d'altération de l'expression d'un gène endogène d'une cellule de vertébré comprenant les étapes consistant à :
a) transfecter la cellule avec une construction d'ADN comprenant :
1) une séquence régulatrice d'ADN exogène qui n'est normalement pas fonctionnellement liée au gène endogène dans la cellule obtenue ;
2) une ou plusieurs séquences ciblantes qui sont homologues à une ou plusieurs séquences d'ADN génomique à un site présélectionné dans la cellule ;
3) une séquence d'ADN amplifiable codant pour un marqueur sélectionnable ; et
4) une séquence codant pour un site donneur d'épissage non apparié ;
b) maintenir la cellule produite dans a) dans des conditions appropriées pour qu'une recombinaison homologue se produise entre les une ou plusieurs séquences d'ADN spécifiées dans a) 2) ci-dessus et les une ou plusieurs séquences génomiques au site présélectionné, de manière à produire une cellule recombinante homologue provenant d'un vertébré ayant les séquences d'ADN de a) 1), a) 3) et a) 4) intégrées dans l'ADN génomique de sorte que la transcription puisse se produire sous le contrôle de la séquence régulatrice de telle manière que le premier exon du gène endogène soit épissuré ;
et
c) cultiver la cellule recombinante homologue produite dans b) dans des conditions qui sélectionnent l'amplification de l'ADN amplifiable codant pour un marqueur sélectionnable et qui permettent l'expression altérée du gène endogène, de telle manière que l'ADN amplifiable codant pour un marqueur sélectionnable et la séquence régulatrice d'ADN exogène fonctionnellement liés à l'ADN du gène endogène soient coamplifiés et que ladite expression altérée soit obtenue.

2. Procédé de la revendication 1, **caractérisé en ce que** la cellule de vertébré est une cellule primaire ou secondaire.

3. Procédé de la revendication 2, **caractérisé en ce que** la cellule primaire ou secondaire est une cellule mammalienne.

4. Procédé de la revendication 2 ou 3, **caractérisé en ce que** la cellule primaire ou secondaire est une cellule humaine.

5. Procédé de l'une quelconque des revendications précédentes, **caractérisé en ce que** la cellule de vertébré est une cellule immortalisée.

6. Procédé selon l'une quelconque des revendications 1 à 5, **caractérisé en ce que** la cellule est choisie dans le groupe constitué de : cellules HT1080, cellules HeLa, cellules de cancer du sein MCF-7, cellules de leucémie K-562, cellules de carcinome KB, cellules de carcinome ovarien 2780AD, cellules Raji, cellules Jurkat, cellules Namalwa, cellules HL-60, cellules Daudi, cellules RPMI 8226, cellules MOLT-4, cellules CHO et cellules de souris.

7. Procédé de l'une quelconque des revendications 1 à 5, **caractérisé en ce que** l'ADN amplifiable codant pour un marqueur sélectionnable code pour un marqueur sélectionnable choisi dans le groupe constitué de : dihydrofolate réductase, adénosine désaminase, et CAD.

8. Procédé selon l'une quelconque des revendications 1 à 7, **caractérisé en ce que** la construction d'ADN comprend en outre (a) un marqueur de sélection positive additionnel, (b) un marqueur de sélection négative, ou (c) un marqueur de sélection positive additionnel et un marqueur de sélection négative.

9. Procédé de la revendication 8 dans lequel le marqueur de sélection positive additionnel est neo.

10. Procédé de la revendication 9 dans lequel le marqueur de sélection positive est neo et le marqueur de sélection négative est gpt ou le gène HSV-TK.

11. Procédé de l'une quelconque des revendications 1 à 10, **caractérisé en ce que** le gène endogène code pour une protéine thérapeutique.

12. Procédé de l'une quelconque des revendications 1 à 11, **caractérisé en ce que** le gène endogène code pour une hormone, une cytokine, un antigène, un anticorps, une enzyme, un facteur de coagulation, une protéine de transport, un récepteur, une protéine régulatrice, une protéine structurale, ou un facteur de transcription.

13. Procédé de l'une quelconque des revendications 1 à 11, **caractérisé en ce que** le gène endogène code pour une protéine thérapeutique choisie dans le groupe constitué du facteur VIII, du facteur IX, de l'érythropoïétine, de l'antitrypsine alpha-1, de la calcitonine, de la glucocérébrosidase, de l'hormone de croissance, du récepteur de lipoprotéine basse densité (LDL), des apolipoprotéines, du récepteur IL-2, d'antagonistes de récepteur IL-2, de l'insuline, de globines, d'immunoglobulines, d'anticorps catalytiques, d'interleukines, de facteurs de croissance similaires à l'insuline, de la superoxyde dismutase, de modifieurs de réponse immunitaire, de la parathormone, d'interférons, de facteurs de croissance nerveuse, d'activateurs tissulaires du plasminogène et de facteurs stimulant les colonies.

14. Procédé de l'une quelconque des revendications 1 à 11, **caractérisé en ce que** le gène endogène code pour une protéine thérapeutique choisie dans le groupe constitué de l'hormone de croissance humaine, de l'insuline humaine, de l'insulinotropine humaine, et de l'érythropoïétine humaine.

15. Procédé selon l'une quelconque des revendications précédentes, **caractérisé en ce que** ladite expression altérée produit une protéine qui n'est pas naturellement exprimée dans la cellule de vertébré par le gène endogène.

16. Procédé selon l'une quelconque des revendications précédentes, **caractérisé en ce que** ladite expression altérée produit le produit protéique naturel du gène endogène après tout traitement post-traduction qui se produit dans la cellule.

17. Procédé selon l'une quelconque des revendications précédentes, **caractérisé en ce que** la séquence régulatrice est choisie dans le groupe constitué de : promoteurs, activateurs, régions de fixation d'échafaudage, éléments régulateurs négatifs, sites d'initiation transcriptionnelle et sites de liaison de protéine régulatrice.

18. Procédé selon l'une quelconque des revendications précédentes, **caractérisé en ce que** la construction comprend une combinaison de séquences régulatrices.

19. Procédé selon l'une quelconque des revendications précédentes, **caractérisé en ce que** la construction comprend un signal de polyadénylation.

20. Cellule recombinante homologue produite par un procédé selon l'une quelconque des revendications 1 à 19, **caractérisée en ce que** ladite cellule comprend un marqueur sélectionnable comme décrit dans la revendication 7.

21. Dispositif contenant une cellule selon la revendication 20 qui exprime un produit thérapeutique, **caractérisé en ce que** le produit thérapeutique, mais pas la cellule, est librement perméable à travers le dispositif.

22. Cellule selon la revendication 20 ou dispositif selon la revendication 21, pour utilisation en médecine.

23. Cellule selon la revendication 20, pour utilisation en thérapie génique.

24. Utilisation d'une cellule selon la revendication 20 dans la préparation d'un dispositif selon la revendication 21.

25. Construction qui est capable d'altérer l'expression d'un gène endogène d'une cellule de vertébré et est adaptée pour utilisation dans un procédé selon l'une quelconque des revendications 1 à 19 ; **caractérisé en ce que** la construction comprend :
1) une séquence régulatrice d'ADN exogène qui n'est normalement pas fonctionnellement liée au gène endogène ;
2) une ou plusieurs séquences ciblantes qui sont homologues à une ou plusieurs séquences d'ADN génomique à un site présélectionné dans la cellule ;
3) une séquence d'ADN amplifiable codant pour un marqueur sélectionnable ; et
4) une séquence codant pour un site donneur d'épissage non apparié ;
**caractérisée en ce que** la construction est telle que, après recombinaison homologue entre lesdites une ou plusieurs séquences ciblantes et lesdites une ou plusieurs séquences génomiques au site présélectionné, la transcription peut se produire sous le contrôle de la séquence régulatrice de telle manière que le premier exon du gène endogène soit épissuré, et où l'ADN amplifiable codant pour le marqueur sélectionnable et la séquence régulatrice d'ADN exogène fonctionnellement liée à l'ADN du gène endogène peut être coamplifié ;
et où ledit marqueur sélectionnable est un marqueur comme décrit dans la revendication 7.

26. Construction selon la revendication 25, pour utilisation en médecine.

27. Construction selon la revendication 25, pour utilisation en thérapie génique.

28. Utilisation d'une construction selon la revendication 25 dans la préparation d'un dispositif selon la revendication 21.
